(19) Europäisches Patentamt
European Patent Office
Office européen des brevets

(11) Veröffentlichungsnummer: **0 277 520 A2**

(12) **EUROPÄISCHE PATENTANMELDUNG**

(21) Anmeldenummer: **88100509.4**

(22) Anmeldetag: **15.01.88**

(51) Int. Cl.4: **C07D 239/26**, C07D 405/12, A01N 43/54

(30) Priorität: **31.01.87 DE 3702962**

(43) Veröffentlichungstag der Anmeldung: **10.08.88 Patentblatt 88/32**

(84) Benannte Vertragsstaaten: **AT BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Elbe, Hans-Ludwig, Dr.**
**Dasnöckel 59**
**D-5600 Wuppertal 11(DE)**
Erfinder: **Dutzmann, Stefan, Dr.**
**Leinenweberweg 33**
**D-4000 Düsseldorf 13(DE)**
Erfinder: **Reinecke, Paul**
**Steinstrasse 8**
**D-5090 Leverkusen 3(DE)**

(54) **Schädlingsbekämpfungsmittel auf Pyrimidin-Derivat-Basis.**

(57) Die Verwendung der teilweise bekannten substituierten Pyrimidine der Formel (I)

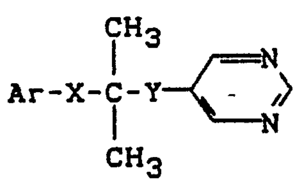

(I)

in welcher
Ar für gegebenenfalls substituiertes Phenyl steht,
X für Sauerstoff, Schwefel, Sulfinyl, Sulfonyl oder eine der Gruppen $-CH_2-$; $-O-CH_2-$; $CH_2-O-$; $-O-CH_2-CH_2-$; $-S(O)_n-CH_2-$; $-CH_2-S(O)_n-$oder $-S(O)_n-CH_2-CH_2-$steht und
Y für eine der Gruppen

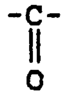 ; 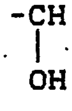 oder 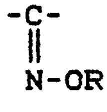

steht, wobei
R für Wasserstoff, Alkyl, Alkenyl, Alkinyl oder für gegenbenenfalls substituiertes Aralkyl steht und
n für eine Zahl 0, 1 oder 2 steht,
zur Bekämpfung von Schädlingen sowie die noch neuen Stoffe und ihre Herstellung, z.B. aus geeigneten Acylenolen mit Formamiden und Formaldehyddimethylacetal oder aus geeigneten Ketonen mit geeigneten Hydroxylaminen.

EP 0 277 520 A2

## Schädlingsbekämpfungsmittel auf Pyrimidin-Derivat Basis

Die Erfindung betrifft die Verwendung von teilweise bekannten Pyrimidin-Verbindungen als Schädlingsbekämpfungsmittel, insbesondere als Fungizide.

Es ist bereits bekannt, daß bestimmte Pyrimidin-Derivate, wie beispielsweise das 5-[1-(4-Chlorbenzyloxy)-2-methyl-prop-1-yl]-pyrimidin oder das 5-[1-(4-Trifluormethoxybenzyloxy)-2,2-dimethyl-prop-1-yl]-pyrimidin fungizide Eigenschaften besitzen (vgl. DE-OS 31 05 374).

Die Wirksamkeit dieser vorbekannten Verbindungen ist jedoch insbesondere bei niedrigen Aufwandmengen und Konzentrationen nicht in allen Anwendungsbereichen völlig zufriedenstellend.

Weiterhin sind bestimmte substituierte Pyrimidine, wie beispielsweise das 2,2-Dimethyl-3-(4-fluorphenyl)-1-(5-pyrimidinyl)-propan-1-on oder das 2,2-Dimethyl-3-(3-chlorphenoxy)-1-(5-pyrimidinyl)-propan-1-on oder das 2,2-Dimethyl-3-(4-chlorphenoxy)-1-(5-pyrimidinyl)-propan-1-on bekannt (vgl. DE-OS 34 31 689). Über eine fungizide Wirksamkeit dieser vorbekannten Pyrimidine ist jedoch nichts bekannt.

Es wurde gefunden, daß die teilweise bekannten substituierten Pyrimidine der allgemeinen Formel (I),

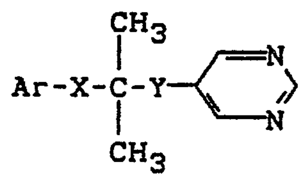
(I)

in welcher

Ar für gegebenenfalls substituiertes Phenyl steht,

X für Sauerstoff, Schwefel, Sulfinyl, Sulfonyl oder eine der Gruppen $-CH_2-$; $-O-CH_2-$; $-CH_2-O-$; $-O-CH_2-CH_2-$; $-S(O)_n-CH_2-$; $-CH_2-S(O)_n-$oder $-S(O)_n-CH_2-CH_2-$steht und

Y für eine der Gruppen

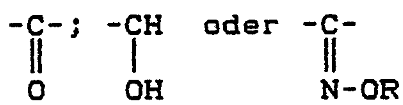

steht, wobei

R für Wasserstoff, Alkyl, Alkenyl, Alkinyl oder für gegebenenfalls substituiertes Aralkyl steht und

n für eine Zahl 0, 1 oder 2 steht,

sowie deren Säureadditionssalze und Metallsalzkomplexe eine gute Wirkung gegen Schädlinge besitzen.

Die Verbindungen der Formel (I) können gegebenenfalls als geometrische und/oder optische Isomere oder Isomerengemische unterschiedlicher Zusammensetzung vorliegen. Sowohl die reinen Isomeren als auch die Isomerengemische werden erfindungsgemäß beansprucht.

Überraschenderweise zeigen die erfindungsgemäß verwendbaren substituierten Pyrimidine der allgemeinen Formel (I) u.a. eine erheblich bessere fungizide Wirksamkeit als die aus dem Stand der Technik als Fungizide bekannten substituierten Pyrimidin-Derivate, wie beispielsweise das 5-[1-(4-Chlorbenzyloxy)-2-methyl-prop-1-yl]-pyrimidin oder das 5-[1-(4-Trifluormethoxybenzyloxy)-2,2-dimethylprop-1-yl]-pyrimidin, welches chemisch und wirkungsmäßig naheliegende Verbindungen sind.

Die erfindungsgemäß verwendbaren substituierten Pyrimidine sind durch die Formel (I) allgemein definiert. Bevorzugt verwendbar sind Verbindungen der Formel (I), bei welchen

Ar für gegebenenfalls einfach bis mehrfach, gleich oder verschieden substituiertes Phenyl steht, wobei als Substituenten infrage kommen: Halogen, Cyano, Nitro sowie jeweils geradkettiges oder verzweigtes Alkyl, Alkoxy, Alkylthio, Alkylsulfinyl oder Alkylsulfonyl mit jeweils 1 bis 4 Kohlenstoffatomen, je weils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen, gegebenenfalls einfach bis mehrfach, gleich oder verschieden durch Halogen substituiertes, zweifach verknüpftes Dioxyalkylen oder jeweils gegebenenfalls einfach bis mehrfach, gleich oder verschieden durch Halogen substituiertes Phenyl oder Phenoxy,

X für Sauerstoff, Schwefel, Sulfinyl, Sulfonyl oder für eine der Gruppen $-CH_2-$; $-O-CH_2-$; $-CH_2-O-$; $-O-CH_2-$

$CH_2$-; -$S(O)_n$-$CH_2$-; -$CH_2$-$S(O)_n$-oder -$S(O)_n$-$CH_2$-$CH_2$-steht und
Y für eine der Gruppen

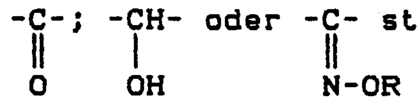 steht,

wobei
R für Wasserstoff oder für jeweils geradkettiges oder verzweigtes Alkyl mit 1 bis 6 Kohlenstoffatomen, Alkenyl mit 3 bis 5 Kohlenstoffatomen oder Alkinyl mit 3 bis 5 Kohlenstoffatomen oder für gegebenenfalls einfach bis mehrfach, gleich oder verschieden substituiertes Benzyl oder Phenylethyl steht, wobei als Substituenten im Phenylteil jeweils die bei dem Rest Ar genannten infrage kommen und
n für eine Zahl 0, 1 oder 2 steht.

Besonders bevorzugt erfindungsgemäß verwendbar sind Verbindungen der Formel (I), bei welchen
Ar für gegebenenfalls ein-bis dreifach, gleich oder verschieden substituiertes Phenyl steht, wobei als Substituenten infrage kommen: Fluor, Chlor, Brom, Cyano, Nitro, Methyl, Ethyl, n-oder i-Propyl, n-, i-, s-oder t-Butyl, Methoxy, Ethoxy, n-oder i-Propoxy, Methylthio, Ethylthio, Trifluormethyl, Dichlorfluormethyl, Difluorchlormethyl, Difluorbrommethyl, Trichlormethyl, Fluormethyl, Difluormethyl, Trifluorethyl, Tetrafluorethyl, Trifluorchlorethyl, Pentafluorethyl, Trifluormethoxy, Difluormethoxy, Fluormethoxy, Dichlorfluormethoxy, Difluorchlormethoxy, Difluorbrommethoxy, Trichlormethoxy, Trifluorethoxy, Tetrafluorethoxy, Pentafluorethoxy, Trifluorchlorethoxy, Trifluordichlorethoxy, Difluortrichlorethoxy, Pentachlorethoxy, Trifluormethylthio, Difluormethylthio, Fluormethylthio, Difluorchlormethylthio, Dichlorfluormethylthio, Difluorethylthio, Difluorbrommethylthio, Trichlormethylthio, Trifluorethylthio, Tetrafluorethylthio, Pentafluorethylthio, Trifluorchlorethylthio, Trifluordichlorethylthio, Pentachlorethylthio, Methyl sulfinyl, Trifluormethylsulfinyl, Dichlorfluormethylsulfinyl, Difluorchlormethylsulfinyl, Fluormethylsulfinyl, Difluormethylsulfinyl, Methylsulfonyl, Trifluormethylsulfonyl, Dichlorfluormethylsulfonyl, Difluorchlormethylsulfonyl, Fluormethylsulfonyl, Difluormethylsulfonyl, Methoxycarbonyl, Ethoxycarbonyl, Methoximinomethyl, Ethoximinomethyl, Methoximinoethyl, Ethoximinoethyl, Difluordioxymethylen, Tetrafluordioxyethylen, Trifluordioxyethylen, Difluordioxyethylen, Dioxymethylen, Dioxyethylen sowie jeweils gegebenenfalls ein-bis dreifach durch Fluor oder Chlor substituiertes Phenyl oder Phenoxy,
X für Sauerstoff, Schwefel, Sulfinyl, Sulfonyl oder für eine der Gruppen -$CH_2$-; -O-$CH_2$-; -$CH_2$-O-; -O-$CH_2$-$CH_2$-; -$S(O)_n$-$CH_2$-; -$CH_2$-$S(O)_n$-oder -$S(O)_n$-$CH_2$-$CH_2$-steht und
Y für eine der Gruppen

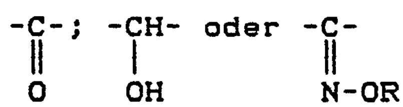

steht, wobei
R für Wasserstoff, Methyl, Ethyl, n-oder i-Propyl, Allyl, Propargyl oder für gegebenenfalls ein-bis dreifach, gleich oder verschieden.substituiertes Benzyl steht, wobei als Substituenten die bei dem Rest Ar genannten infrage kommen und
n für eine Zahl 0, 1 oder 2 steht.

Bevorzugte erfindungsgemäß verwendbare Verbindungen sind auch Additionsprodukte aus Säuren und denjenigen substituierten Pyrimidinen der Formel (I), in denen die Substituenten Ar, X und Y die Bedeutung haben, die bereits vorzugsweise für diese Substituenten genannt wurden.

Zu den Säuren, die addiert werden können, gehören vorzugsweise Halogenwasserstoffsäuren, wie z.B. Chlorwasserstoffsäure und Bromwasserstoffsäure, insbesondere Chlorwasserstoffsäure, ferner Phosphorsäure, Salpetersäure, mono-, bi-und trifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure und Milchsäure sowie Sulfonsäuren, wie p-Toluolsulfonsäure und 1,5-Naphthalindisulfonsäure.

Außerdem bevorzugte erfindungsgemäß verwendbare Verbindungen sind Additionsprodukte aus Salzen von Metallen der II. bis IV. Haupt-und der I. und II. sowie IV. bis VIII. Nebengruppe und denjenigen substituierten Pyrimidinen der Formel (I), in denen die Substituenten Ar, X und Y die Bedeutungen haben, die bereits vorzugsweise für diese Substituenten genannt wurden.

Hierbei sind Salze des Kupfers, Zinks, Mangans, Magnesiums, Zinns, Eisens und des Nickels besonders bevor zugt. Als Anionen dieser Salze kommen solche in Betracht, die sich von solchen Säuren

ableiten, die zu physiologisch verträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, ferner Phosphorsäure, Salpetersäure und Schwefelsäure.

Im einzelnen seien außer den bei den Herstellungsbeispielen genannten Verbindung die folgenden substituierten Pyrimidine der allgemeinen Formel (I) genannt:

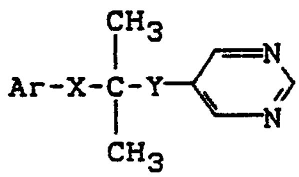 (I)

| Ar | X | Y | X | Y |
|---|---|---|---|---|
| 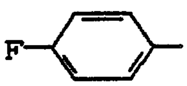 | O | 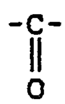 | O | 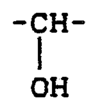 |
| 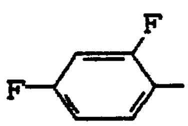 | O | 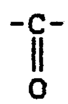 | O | 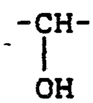 |
| 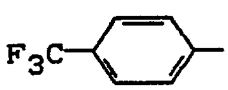 | O | 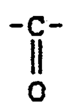 | O | 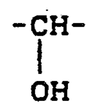 |

| Ar | X | Y | X | Y |
|---|---|---|---|---|
| 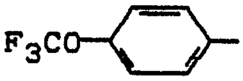 | O | 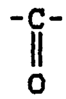 | O | 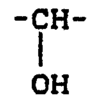 |
| 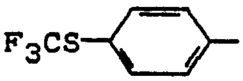 | O | 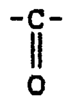 | O | 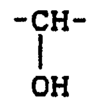 |
| 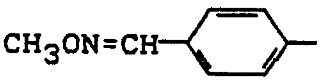 | O | 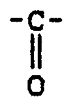 | O | 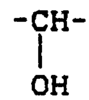 |
| 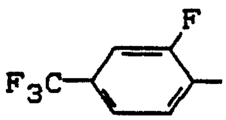 | O | 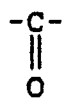 | O | 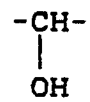 |
| 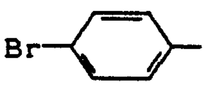 | O | 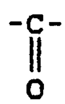 | O | 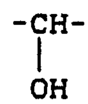 |
| 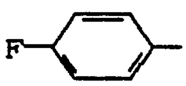 | S | 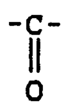 | S | 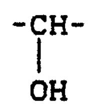 |
| 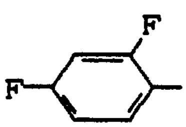 | S | 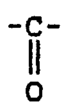 | S | 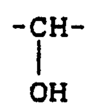 |
| 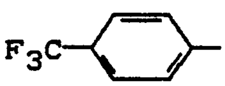 | S | 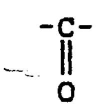 | S | 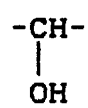 |

| Ar | X | Y | X | Y |
|---|---|---|---|---|
| 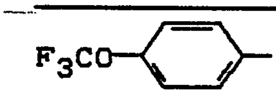 | S | 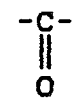 | S | 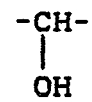 |
| 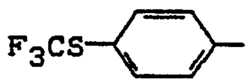 | S | 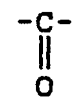 | S | 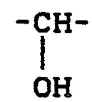 |
| 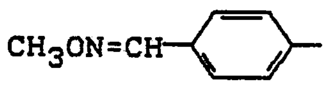 | S | 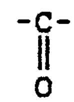 | S | 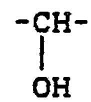 |
| 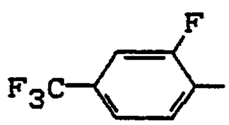 | S | 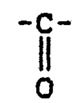 | S | 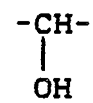 |
| 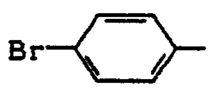 | S | 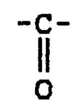 | S | 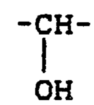 |
| 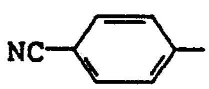 | O | 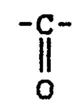 | O | 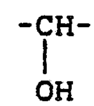 |
| 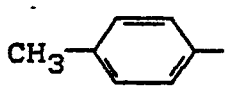 | O | 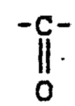 | O | 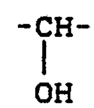 |
| 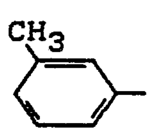 | O | 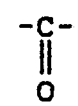 | O | 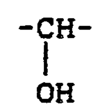 |

| Ar | X | Y | X | Y |
|---|---|---|---|---|
| 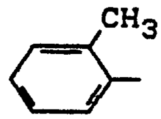 | O | 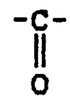 | O | 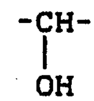 |
| 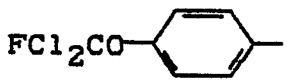 | O | 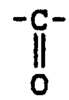 | O | 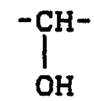 |
| 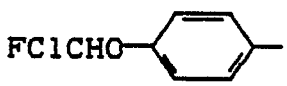 | O | 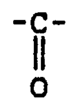 | O | 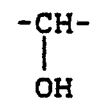 |
| 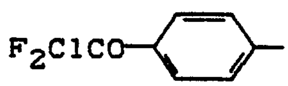 | O | 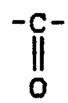 | O | 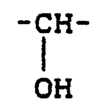 |
| 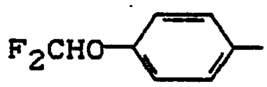 | O | 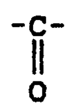 | O | 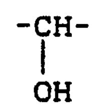 |
| 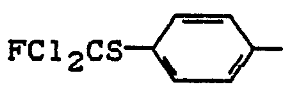 | O | 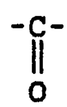 | O | 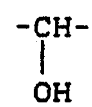 |
| 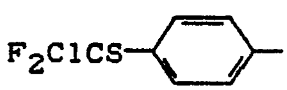 | O | 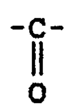 | O | 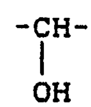 |
| 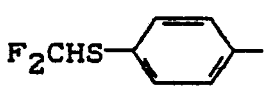 | O | 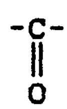 | O | 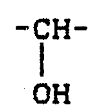 |

| Ar | X | Y | X | Y |
|---|---|---|---|---|
| 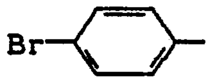 | $-CH_2-O-$ | 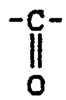 | $-CH_2-O-$ | 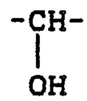 |
| 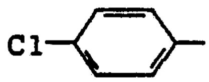 | $-CH_2-O-$ | 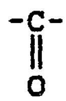 | $-CH_2-O-$ | 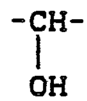 |
| 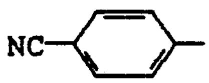 | S | 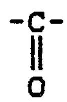 | S | 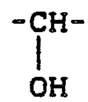 |
| 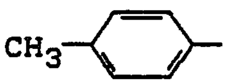 | S | 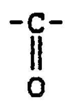 | S | 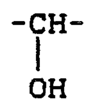 |
| 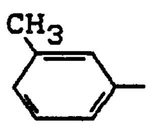 | S | 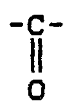 | S | 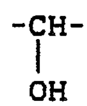 |
| 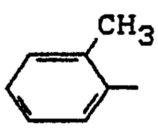 | S | 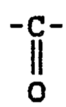 | S | 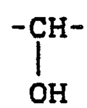 |
| 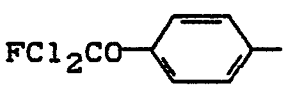 | S | 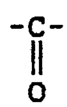 | S | 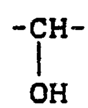 |
| 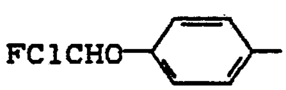 | S | 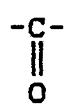 | S | 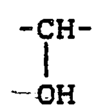 |

| Ar | X | Y | X | Y |
|---|---|---|---|---|
| 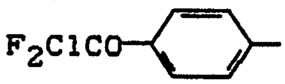 | S | -C-<br>‖<br>O | S | -CH-<br>\|<br>OH |
| 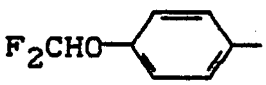 | S | -C-<br>‖<br>O | S | -CH-<br>\|<br>OH |
| 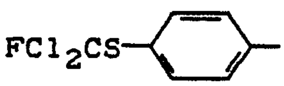 | S | -C-<br>‖<br>O | S | -CH-<br>\|<br>OH |
| 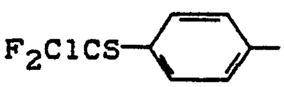 | S | -C-<br>‖<br>O | S | -CH-<br>\|<br>OH |
| 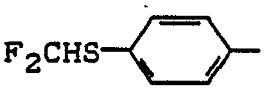 | S | -C-<br>‖<br>O | S | -CH-<br>\|<br>OH |
| 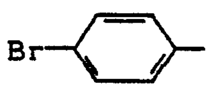 | $-CH_2-O-$ | -C-<br>‖<br>N-OH | $-CH_2-O-$ | -C-<br>‖<br>$N-OCH_3$ |
| 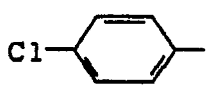 | $-CH_2-O-$ | -C-<br>‖<br>N-OH | $-CH_2-O-$ | -C-<br>‖<br>$N-OCH_3$ |
| 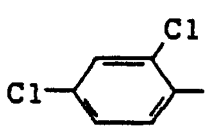 | $-CH_2-O-$ | -C-<br>‖<br>O | $-CH_2-O-$ | -CH-<br>\|<br>OH |
| 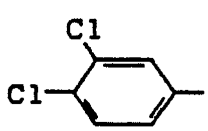 | $-CH_2-O-$ | -C-<br>‖<br>O | $-CH_2-O-$ | -CH-<br>\|<br>OH |

| Ar | X | Y | X | Y |
|---|---|---|---|---|
| 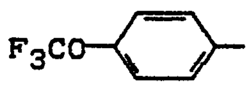 | $-CH_2-O-$ | -C-<br>‖<br>O | $-CH_2-O-$ | -CH-<br>│<br>OH |
| 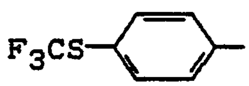 | $-CH_2-O-$ | -C-<br>‖<br>O | $-CH_2-O-$ | -CH-<br>│<br>OH |
| 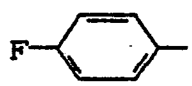 | $-CH_2-O-$ | -C-<br>‖<br>O | $-CH_2-O-$ | -CH-<br>│<br>OH |
| 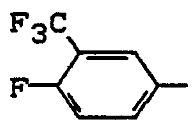 | $-CH_2-O-$ | -C-<br>‖<br>O | $-CH_2-O-$ | -CH-<br>│<br>OH |
| 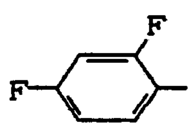 | $-CH_2-O-$ | -C-<br>‖<br>O | $-CH_2-O-$ | -CH-<br>│<br>OH |
| 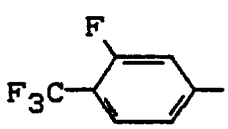 | $-CH_2-$ | -C-<br>‖<br>O | $-CH_2-$ | -CH-<br>│<br>OH |
| 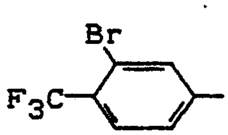 | $-CH_2-$ | -C-<br>‖<br>O | $-CH_2-$ | -CH-<br>│<br>OH |
| 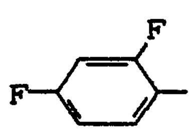 | $-CH_2-$ | -C-<br>‖<br>O | $-CH_2-$ | -CH-<br>│<br>OH |

| Ar | X | Y | X | Y |
|---|---|---|---|---|
| 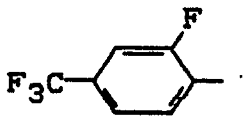 | $-CH_2-$ | 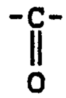 | $-CH_2-$ | 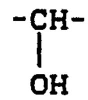 |
| 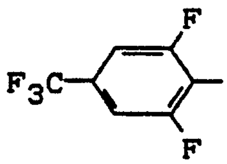 | $-CH_2-$ | 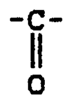 | $-CH_2-$ | 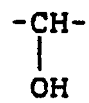 |
| 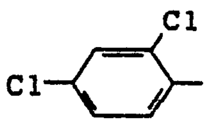 | $-CH_2-O-$ | 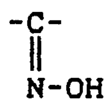 | $-CH_2-O-$ | 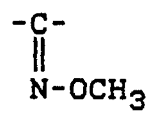 |
| 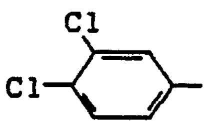 | $-CH_2-O-$ | 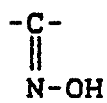 | $-CH_2-O-$ | 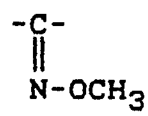 |
| 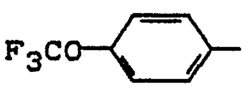 | $-CH_2-O-$ | 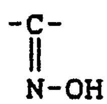 | $-CH_2-O-$ | 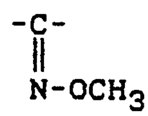 |
| 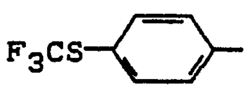 | $-CH_2-O-$ | 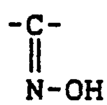 | $-CH_2-O-$ | 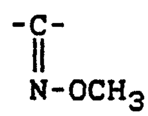 |
| 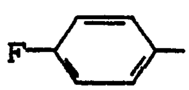 | $-CH_2-O-$ | 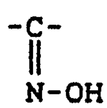 | $-CH_2-O-$ | 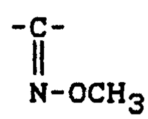 |
| 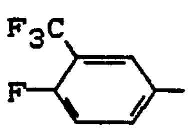 | $-CH_2-O-$ | 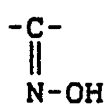 | $-CH_2-O-$ | 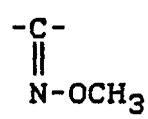 |

| Ar | X | Y | X | Y |
|---|---|---|---|---|
| 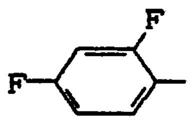 | $-CH_2-O-$ | 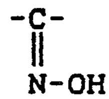 | $-CH_2-O-$ | 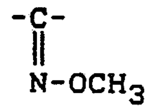 |
| 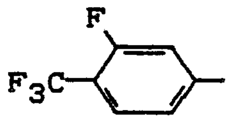 | $-CH_2-$ | 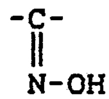 | $-CH_2-$ | 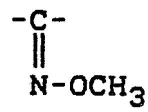 |
| 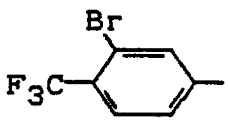 | $-CH_2-$ | 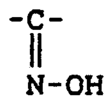 | $-CH_2-$ | 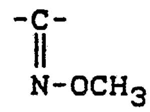 |
| 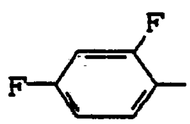 | $-CH_2-$ | 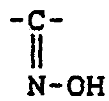 | $-CH_2-$ | 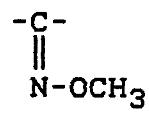 |
| 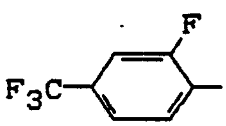 | $-CH_2-$ | 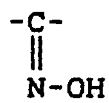 | $-CH_2-$ | 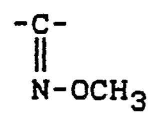 |
| 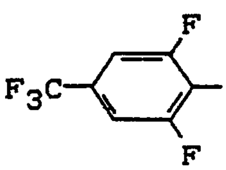 | $-CH_2-$ | 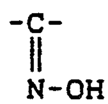 | $-CH_2-$ | 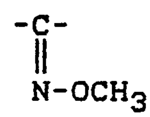 |
| 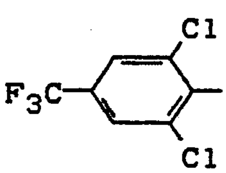 | $-CH_2-$ | 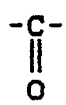 | $-CH_2-$ | 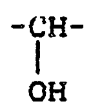 |

| Ar | X | Y | X | Y |
|---|---|---|---|---|
| 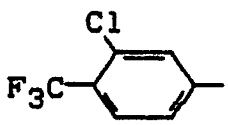 | $-CH_2-$ | 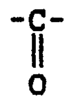 | $-CH_2-$ | 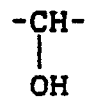 |
| 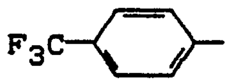 | $-CH_2-$ | 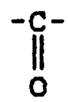 | $-CH_2-$ | 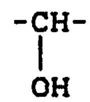 |
| 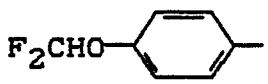 | $-CH_2-$ | 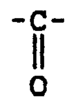 | $-CH_2-$ | 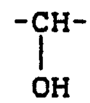 |
| 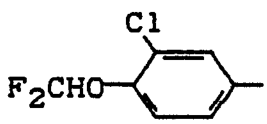 | $-CH_2-$ | 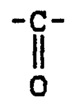 | $-CH_2-$ | 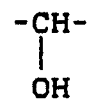 |
| 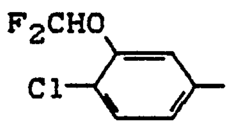 | $-CH_2-$ | 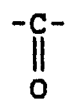 | $-CH_2-$ | 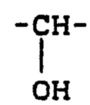 |
| 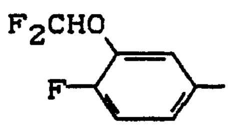 | $-CH_2-$ | 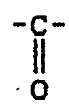 | $-CH_2-$ | 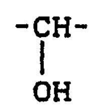 |
| 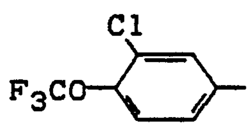 | $-CH_2-$ | 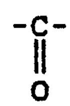 | $-CH_2-$ | 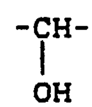 |
| 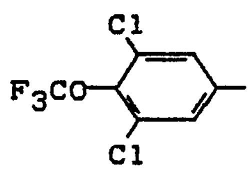 | $-CH_2-$ | 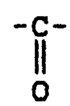 | $-CH_2-$ | 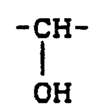 |

| Ar | X | Y | X | Y |
|---|---|---|---|---|
| 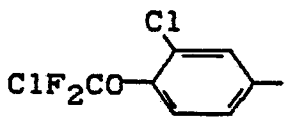 | $-CH_2-$ | -C-<br>‖<br>O | $-CH_2-$ | -CH-<br>\|<br>OH |
| 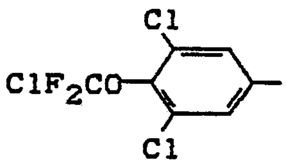 | $-CH_2-$ | -C-<br>‖<br>O | $-CH_2-$ | -CH-<br>\|<br>OH |
| 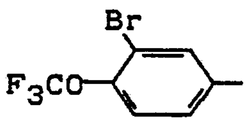 | $-CH_2-$ | -C-<br>‖<br>O | $-CH_2-$ | -CH-<br>\|<br>OH |
| 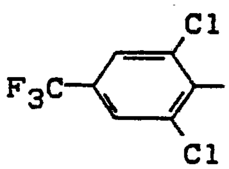 | $-CH_2-$ | -C-<br>‖<br>N-OH | $-CH_2-$ | -C-<br>‖<br>$N-OCH_3$ |
| 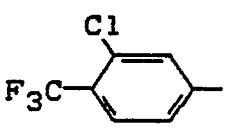 | $-CH_2-$ | -C-<br>‖<br>N-OH | $-CH_2-$ | -C-<br>‖<br>$N-OCH_3$ |
| 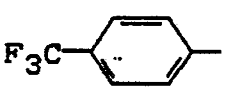 | $-CH_2-$ | -C-<br>‖<br>N-OH | $-CH_2-$ | -C-<br>‖<br>$N-OCH_3$ |
| 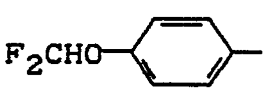 | $-CH_2-$ | -C-<br>‖<br>N-OH | $-CH_2-$ | -C-<br>‖<br>$N-OCH_3$ |
| 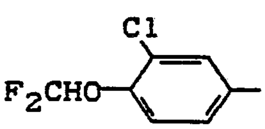 | $-CH_2-$ | -C-<br>‖<br>N-OH | $-CH_2-$ | -C-<br>‖<br>$N-OCH_3$ |

| Ar | X | Y | X | Y |
|---|---|---|---|---|
| 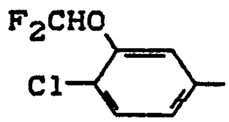 | $-CH_2-$ | -C-<br>‖<br>N-OH | $-CH_2-$ | -C-<br>‖<br>$N-OCH_3$ |
| 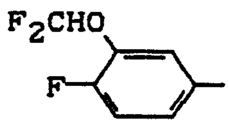 | $-CH_2-$ | -C-<br>‖<br>N-OH | $-CH_2-$ | -C-<br>‖<br>$N-OCH_3$ |
| 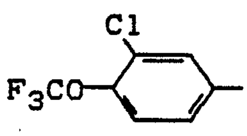 | $-CH_2-$ | -C-<br>‖<br>N-OH | $-CH_2-$ | -C-<br>‖<br>$N-OCH_3$ |
| 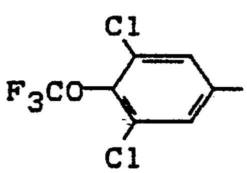 | $-CH_2-$ | -C-<br>‖<br>N-OH | $-CH_2-$ | -C-<br>‖<br>$N-OCH_3$ |
| 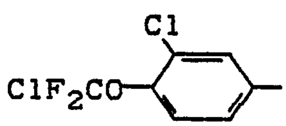 | $-CH_2-$ | -C-<br>‖<br>N-OH | $-CH_2-$ | -C-<br>‖<br>$N-OCH_3$ |
| 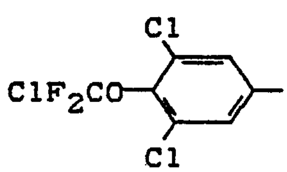 | $-CH_2-$ | -C-<br>‖<br>N-OH | $-CH_2-$ | -C-<br>‖<br>$N-OCH_3$ |
| 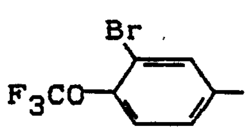 | $-CH_2-$ | -C-<br>‖<br>N-OH | $-CH_2-$ | -C-<br>‖<br>$N-OCH_3$ |

| Ar | X | Y | X | Y |
|---|---|---|---|---|
| 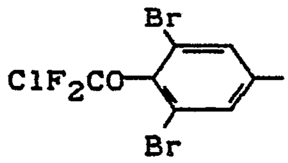 | $-CH_2-$ | 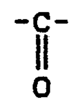 | $-CH_2-$ | 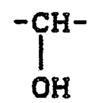 |
| 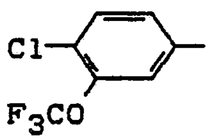 | $-CH_2-$ | 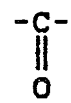 | $-CH_2-$ | 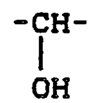 |
| 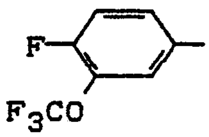 | $-CH_2-$ | 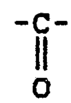 | $-CH_2-$ | 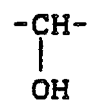 |
| 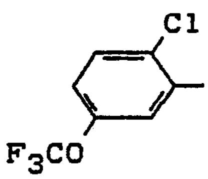 | $-CH_2-$ | 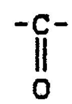 | $-CH_2-$ | 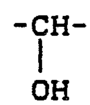 |
| 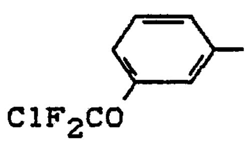 | $-CH_2-$ | 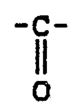 | $-CH_2-$ | 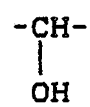 |
| 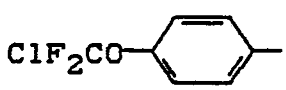 | $-CH_2-$ | 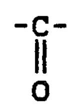 | $-CH_2-$ | 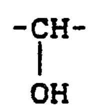 |
| 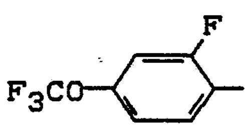 | $-CH_2-$ | 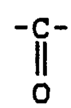 | $-CH_2-$ | 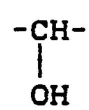 |
| 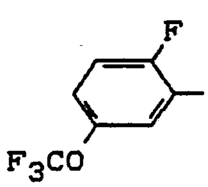 | $-CH_2-$ | 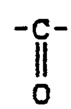 | $-CH_2-$ | 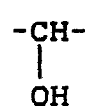 |

| Ar | X | Y | X | Y |
|---|---|---|---|---|
| 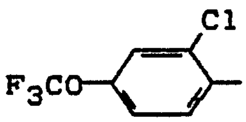 | $-CH_2-$ | -C-<br>‖<br>O | $-CH_2-$ | -CH-<br>\|<br>OH |
| 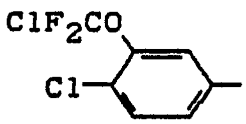 | $-CH_2-$ | -C-<br>‖<br>O | $-CH_2-$ | -CH-<br>\|<br>OH |
| 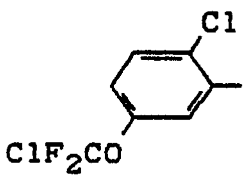 | $-CH_2-$ | -C-<br>‖<br>O | $-CH_2-$ | -CH-<br>\|<br>OH |
| 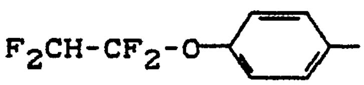 | $-CH_2-$ | -C-<br>‖<br>O | $-CH_2-$ | -CH-<br>\|<br>OH |
| 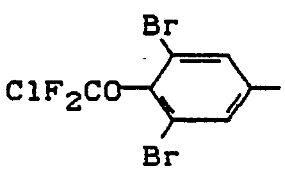 | $-CH_2-$ | -C-<br>‖<br>N-OH | $-CH_2-$ | -C-<br>‖<br>$N-OCH_3$ |
| 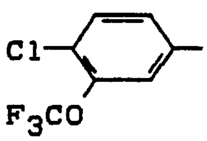 | $-CH_2-$ | -C-<br>‖<br>N-OH | $-CH_2-$ | -C-<br>‖<br>$N-OCH_3$ |
| 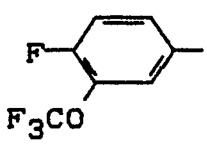 | $-CH_2-$ | -C-<br>‖<br>N-OH | $-CH_2-$ | -C-<br>‖<br>$N-OCH_3$ |
| 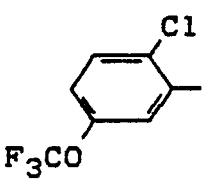 | $-CH_2-$ | -C-<br>‖<br>N-OH | $-CH_2-$ | -C-<br>‖<br>$N-OCH_3$ |

| Ar | X | Y | X | Y |
|---|---|---|---|---|
| 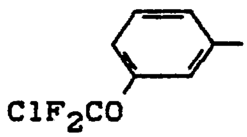 | $-CH_2-$ | -C-<br>‖<br>N-OH | $-CH_2-$ | -C-<br>‖<br>$N-OCH_3$ |
| 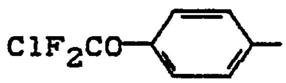 | $-CH_2-$ | -C-<br>‖<br>N-OH | $-CH_2-$ | -C-<br>‖<br>$N-OCH_3$ |
| 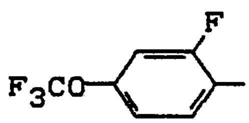 | $-CH_2-$ | -C-<br>‖<br>N-OH | $-CH_2-$ | -C-<br>‖<br>$N-OCH_3$ |
| 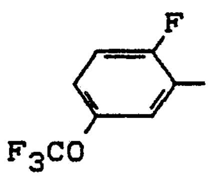 | $-CH_2-$ | -C-<br>‖<br>N-OH | $-CH_2-$ | -C-<br>‖<br>$N-OCH_3$ |
| 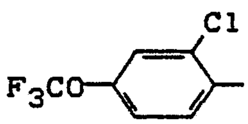 | $-CH_2-$ | -C-<br>‖<br>N-OH | $-CH_2-$ | -C-<br>‖<br>$N-OCH_3$ |
| 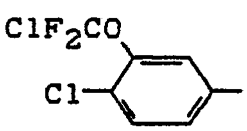 | $-CH_2-$ | -C-<br>‖<br>N-OH | $-CH_2-$ | -C-<br>‖<br>$N-OCH_3$ |
| 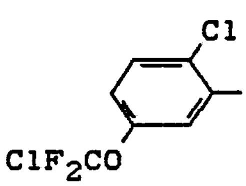 | $-CH_2-$ | -C-<br>‖<br>N-OH | $-CH_2-$ | -C-<br>‖<br>$N-OCH_3$ |
| 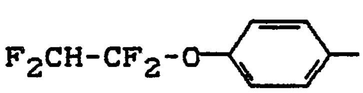 | $-CH_2-$ | -C-<br>‖<br>N-OH | $-CH_2-$ | -C-<br>‖<br>$N-OCH_3$ |

| Ar | X | Y | X | Y |
|---|---|---|---|---|
| 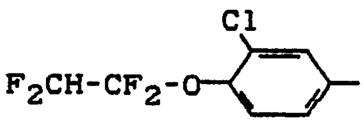 | $-CH_2-$ | -C-<br>‖<br>O | $-CH_2-$ | -CH-<br>\|<br>OH |
| 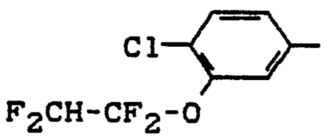 | $-CH_2-$ | -C-<br>‖<br>O | $-CH_2-$ | -CH-<br>\|<br>OH |
| 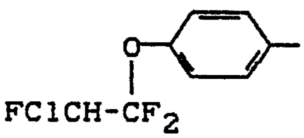 | $-CH_2-$ | -C-<br>‖<br>O | $-CH_2-$ | -CH-<br>\|<br>OH |
| 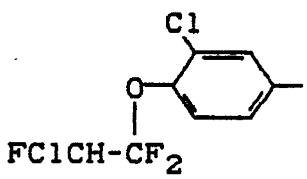 | $-CH_2-$ | -C-<br>‖<br>O | $-CH_2-$ | -CH-<br>\|<br>OH |
| 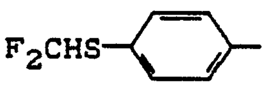 | $-CH_2-$ | -C-<br>‖<br>O | $-CH_2-$ | -CH-<br>\|<br>OH |
| 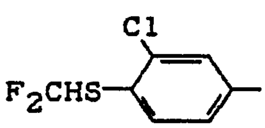 | $-CH_2-$ | -C-<br>‖<br>O | $-CH_2-$ | -CH-<br>\|<br>OH |
| 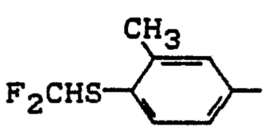 | $-CH_2-$ | -C-<br>‖<br>O | $-CH_2-$ | -CH-<br>\|<br>OH |
| 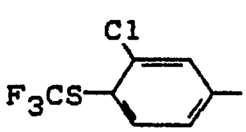 | $-CH_2-$ | -C-<br>‖<br>O | $-CH_2-$ | -CH-<br>\|<br>OH |

| Ar | X | Y | X | Y |
|---|---|---|---|---|
| 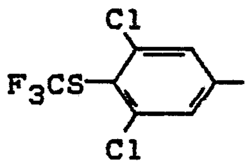 | $-CH_2-$ | 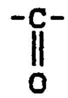 | $-CH_2-$ | 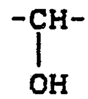 |
| 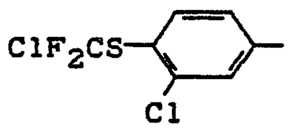 | $-CH_2-$ | 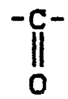 | $-CH_2-$ | 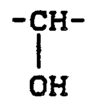 |
| 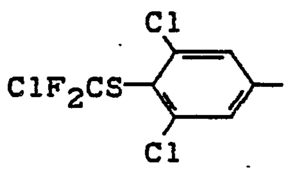 | $-CH_2-$ | 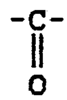 | $-CH_2-$ | 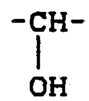 |
| 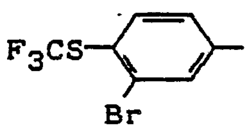 | $-CH_2-$ | 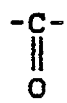 | $-CH_2-$ | 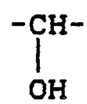 |
| 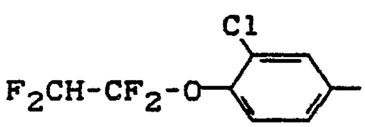 | $-CH_2-$ | 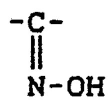 | $-CH_2-$ | 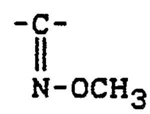 |
| 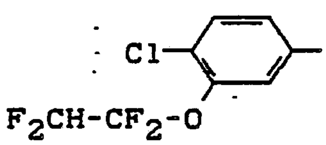 | $-CH_2-$ | 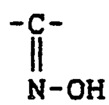 | $-CH_2-$ | 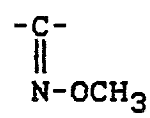 |
| 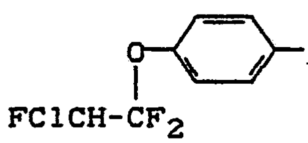 | $-CH_2-$ | 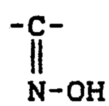 | $-CH_2-$ | 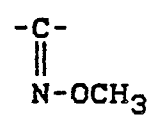 |
| 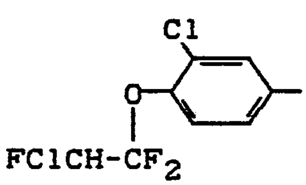 | $-CH_2-$ | 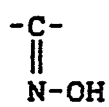 | $-CH_2-$ | 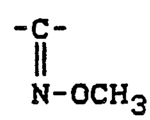 |

| Ar | X | Y | X | Y |
|---|---|---|---|---|
| 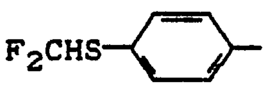 | $-CH_2-$ | -C-<br>‖<br>N-OH | $-CH_2-$ | -C-<br>‖<br>$N-OCH_3$ |
| 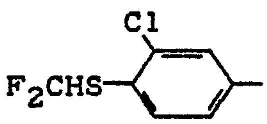 | $-CH_2-$ | -C-<br>‖<br>N-OH | $-CH_2-$ | -C-<br>‖<br>$N-OCH_3$ |
| 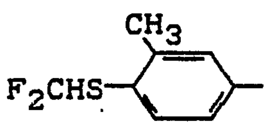 | $-CH_2-$ | -C-<br>‖<br>N-OH | $-CH_2-$ | -C-<br>‖<br>$N-OCH_3$ |
| 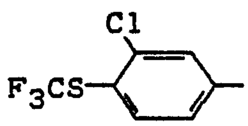 | $-CH_2-$ | -C-<br>‖<br>N-OH | $-CH_2-$ | -C-<br>‖<br>$N-OCH_3$ |
| 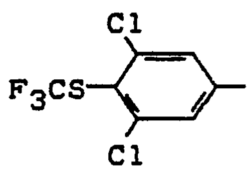 | $-CH_2-$ | -C-<br>‖<br>N-OH | $-CH_2-$ | -C-<br>‖<br>$N-OCH_3$ |
| 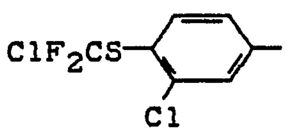 | $-CH_2-$ | -C-<br>‖<br>N-OH | $-CH_2-$ | -C-<br>‖<br>$N-OCH_3$ |
| 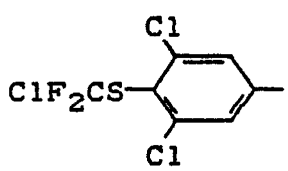 | $-CH_2-$ | -C-<br>‖<br>N-OH | $-CH_2-$ | -C-<br>‖<br>$N-OCH_3$ |
| 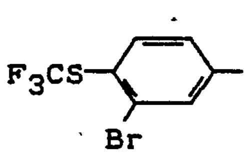 | $-CH_2-$ | -C-<br>‖<br>N-OH | $-CH_2-$ | -C-<br>‖<br>$N-OCH_3$ |

| Ar | X | Y | X | Y |
|---|---|---|---|---|
| 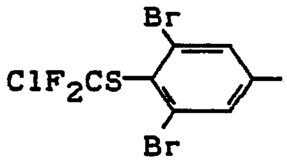 | $-CH_2-$ | $-C(=O)-$ | $-CH_2-$ | $-CH(OH)-$ |
| 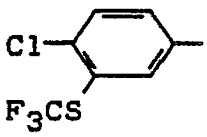 | $-CH_2-$ | $-C(=N-OH)-$ | $-CH_2-$ | $-C(=N-OCH_3)-$ |
| 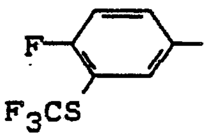 | $-CH_2-$ | $-C(=N-OH)-$ | $-CH_2-$ | $-C(=N-OCH_3)-$ |
| 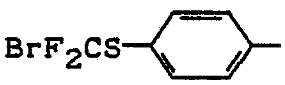 | $-CH_2-$ | $-C(=N-OH)-$ | $-CH_2-$ | $-C(=N-OCH_3)-$ |
| 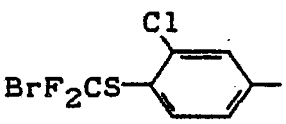 | $-CH_2-$ | $-C(=N-OH)-$ | $-CH_2-$ | $-C(=N-OCH_3)-$ |
| 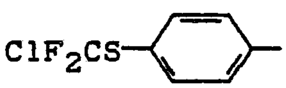 | $-CH_2-$ | $-C(=N-OH)-$ | $-CH_2-$ | $-C(=N-OCH_3)-$ |
| 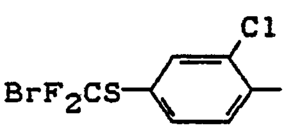 | $-CH_2-$ | $-C(=N-OH)-$ | $-CH_2-$ | $-C(=N-OCH_3)-$ |
| 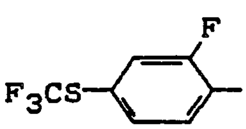 | $-CH_2-$ | $-C(=N-OH)-$ | $-CH_2-$ | $-C(=N-OCH_3)-$ |

| Ar | X | Y | X | Y |
|---|---|---|---|---|
| 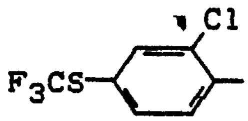 | $-CH_2-$ | -C-<br>‖<br>N-OH | $-CH_2-$ | -C-<br>‖<br>$N-OCH_3$ |
| 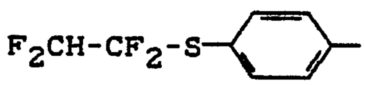 | $-CH_2-$ | -C-<br>‖<br>N-OH | $-CH_2-$ | -C-<br>‖<br>$N-OCH_3$ |
| 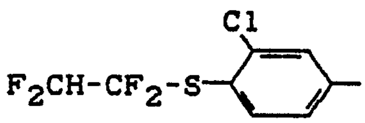 | $-CH_2-$ | -C-<br>‖<br>N-OH | $-CH_2-$ | -C-<br>‖<br>$N-OCH_3$ |
| 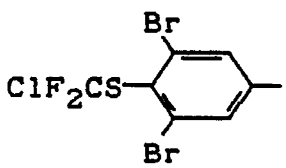 | $-CH_2-$ | -C-<br>‖<br>N-OH | $-CH_2-$ | -C-<br>‖<br>$N-OCH_3$ |
| 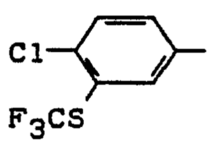 | $-CH_2-$ | -C-<br>‖<br>N-OH | $-CH_2-$ | -C-<br>‖<br>$N-OCH_3$ |
| 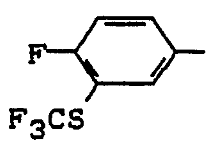 | $-CH_2-$ | -C-<br>‖<br>N-OH | $-CH_2-$ | -C-<br>‖<br>$N-OCH_3$ |
| 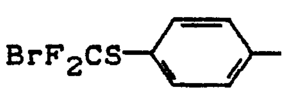 | $-CH_2-$ | -C-<br>‖<br>N-OH | $-CH_2-$ | -C-<br>‖<br>$N-OCH_3$ |
| 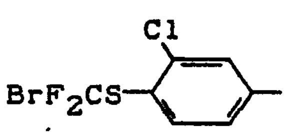 | $-CH_2-$ | -C-<br>‖<br>N-OH | $-CH_2-$ | -C-<br>‖<br>$N-OCH_3$ |

| Ar | X | Y | X | Y |
|---|---|---|---|---|
| 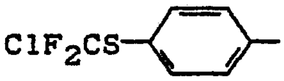 | $-CH_2-$ | 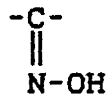 | $-CH_2-$ | 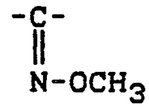 |
| 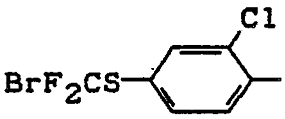 | $-CH_2-$ | 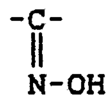 | $-CH_2-$ | 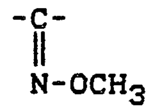 |
| 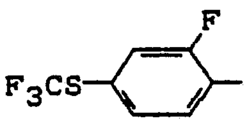 | $-CH_2-$ | 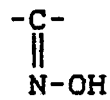 | $-CH_2-$ | 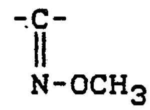 |
| 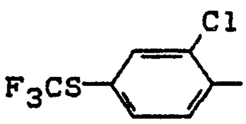 | $-CH_2-$ | 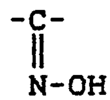 | $-CH_2-$ | 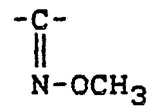 |
| 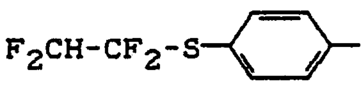 | $-CH_2-$ | 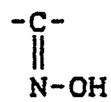 | $-CH_2-$ | 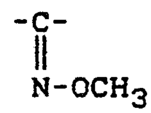 |
| 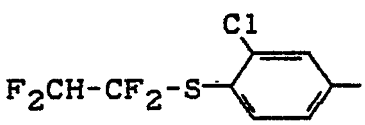 | $-CH_2-$ | 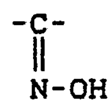 | $-CH_2-$ | 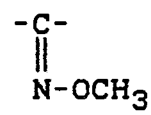 |
| 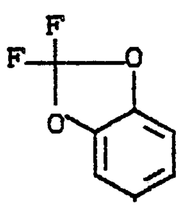 | $-CH_2-$ | 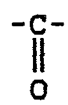 | $-CH_2-$ | 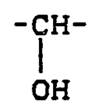 |
| 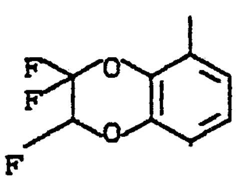 | $-CH_2-$ | 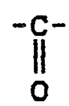 | $-CH_2-$ | 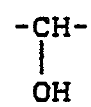 |

| Ar | X | Y | X | Y |
|---|---|---|---|---|
| 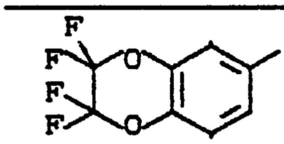 | $-CH_2-$ | -C-<br>‖<br>O | $-CH_2-$ | -CH-<br>\|<br>OH |
| 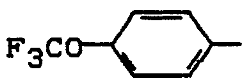 | $-O-CH_2-$ | -C-<br>‖<br>O | $-O-CH_2-$ | -CH-<br>\|<br>OH |
| 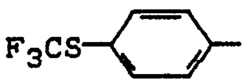 | $-O-CH_2-$ | -C-<br>‖<br>O | $-O-CH_2-$ | -CH-<br>\|<br>OH |
| 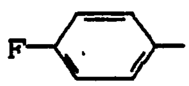 | $-O-CH_2-$ | -C-<br>‖<br>O | $-O-CH_2-$ | -CH-<br>\|<br>OH |
| 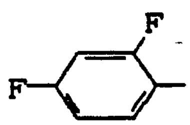 | $-O-CH_2-$ | -C-<br>‖<br>O | $-O-CH_2-$ | -CH-<br>\|<br>OH |
| 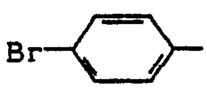 | $-O-CH_2-$ | -C-<br>‖<br>O | $-O-CH_2-$ | -CH-<br>\|<br>OH |
| 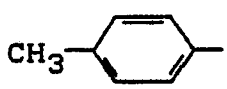 | $-O-CH_2-$ | -C-<br>‖<br>O | $-O-CH_2-$ | -CH-<br>\|<br>OH |
| 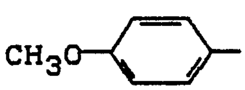 | $-O-CH_2-$ | -C-<br>‖<br>O | $-O-CH_2-$ | -CH-<br>\|<br>OH |
| 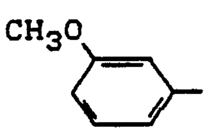 | $-O-CH_2-$ | -C-<br>‖<br>O | $-O-CH_2-$ | -CH-<br>\|<br>OH |

| Ar | X | Y | X | Y |
|---|---|---|---|---|
| 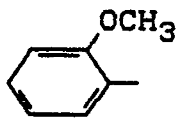 | -O-$CH_2$- | -C-<br>‖<br>O | -O-$CH_2$- | -CH-<br>\|<br>OH |
| 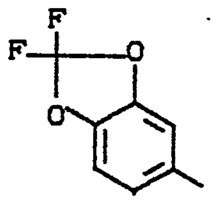 | -$CH_2$- | -C-<br>‖<br>N-OH | -$CH_2$- | -C-<br>‖<br>N-$OCH_3$ |
| 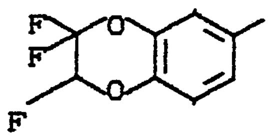 | -$CH_2$- | -C-<br>‖<br>N-OH | -$CH_2$- | -C-<br>‖<br>N-$OCH_3$ |
| 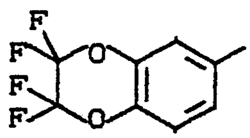 | -$CH_2$- | -C-<br>‖<br>N-OH | -$CH_2$- | -C-<br>‖<br>N-$OCH_3$ |
| 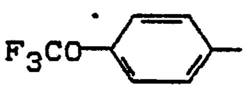 | -S-$CH_2$- | -C-<br>‖<br>O | -S-$CH_2$- | -CH-<br>\|<br>OH |
| 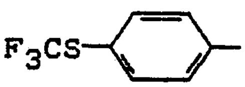 | -S-$CH_2$- | -C-<br>‖<br>O | -S-$CH_2$- | -CH-<br>\|<br>OH |
| 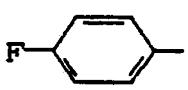 | -S-$CH_2$- | -C-<br>‖<br>O | -S-$CH_2$- | -CH-<br>\|<br>OH |
| 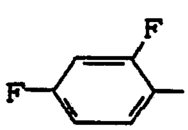 | -S-$CH_2$- | -C-<br>‖<br>O | -S-$CH_2$- | -CH-<br>\|<br>OH |

| Ar | X | Y | X | Y |
|---|---|---|---|---|
| 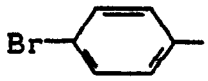 | $-S-CH_2-$ | 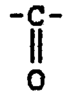 | $-S-CH_2-$ | 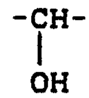 |
| 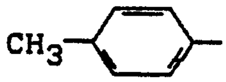 | $-S-CH_2-$ | 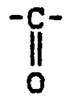 | $-S-CH_2-$ | 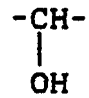 |
| 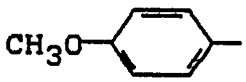 | $-S-CH_2-$ | 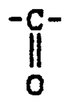 | $-S-CH_2-$ | 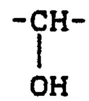 |
| 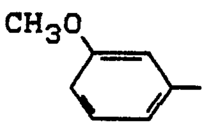 | $-S-CH_2-$ | 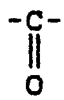 | $-S-CH_2-$ | 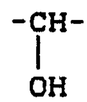 |
| 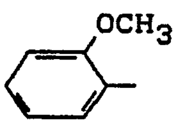 | $-S-CH_2-$ | 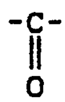 | $-S-CH_2-$ | 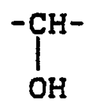 |
| 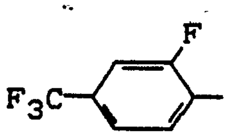 | $-O-CH_2-$ | 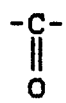 | $-S-CH_2-$ | 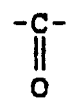 |
| 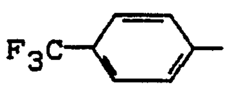 | $-O-CH_2-$ | 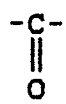 | $-S-CH_2-$ | 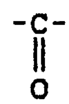 |
| 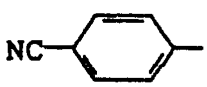 | $-O-CH_2-$ | 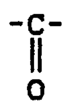 | $-S-CH_2-$ | 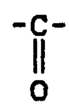 |

| Ar | X | Y | X | Y |
|---|---|---|---|---|
| 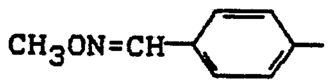 | $-O-CH_2-$ | 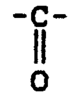 | $-S-CH_2-$ | 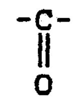 |
| 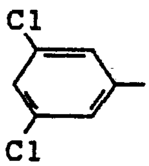 | $-O-CH_2-$ | 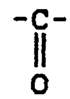 | $-S-CH_2-$ | 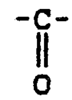 |
| 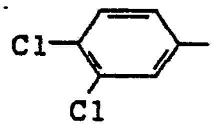 | $-O-CH_2-$ | 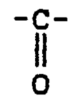 | $-S-CH_2-$ | 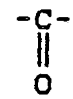 |
| 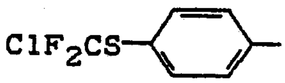 | $-O-CH_2-$ | 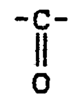 | $-S-CH_2-$ | 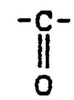 |
| 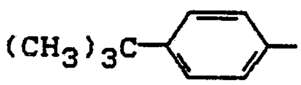 | $-O-CH_2-$ | 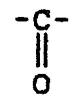 | $-S-CH_2-$ | 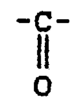 |
| 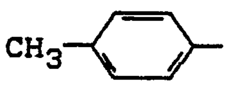 | $-O-CH_2-$ | 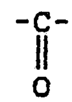 | $-S-CH_2-$ | 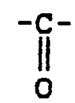 |
| 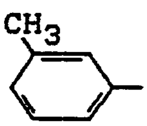 | $-O-CH_2-$ | 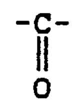 | $-S-CH_2-$ | 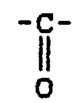 |
| 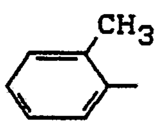 | $-O-CH_2-$ | 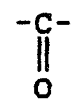 | $-S-CH_2-$ | 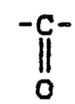 |

| Ar | X | Y | X | Y |
|---|---|---|---|---|
| 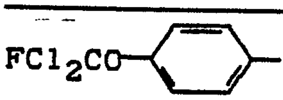 | -O-CH$_2$- | -C-<br>O | -S-CH$_2$- | -C-<br>O |
| 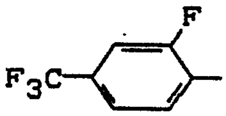 | -O-CH$_2$- | -CH-<br>OH | -S-CH$_2$- | -CH-<br>OH |
| 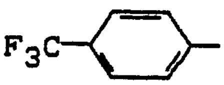 | -O-CH$_2$- | -CH-<br>OH | -S-CH$_2$- | -CH-<br>OH |
| 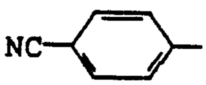 | -O-CH$_2$- | -CH-<br>OH | -S-CH$_2$- | -CH-<br>OH |
| 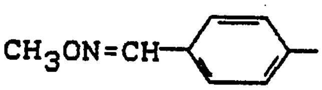 | -O-CH$_2$- | -CH-<br>OH | -S-CH$_2$- | -CH-<br>OH |
| 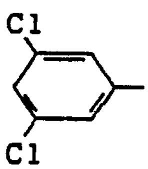 | -O-CH$_2$- | -CH-<br>OH | -S-CH$_2$- | -CH-<br>OH |
| 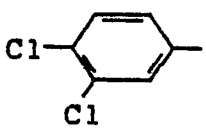 | -O-CH$_2$- | -CH-<br>OH | -S-CH$_2$- | -CH-<br>OH |
| 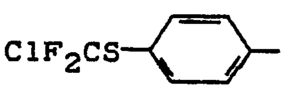 | -O-CH$_2$- | -CH-<br>OH | -S-CH$_2$- | -CH-<br>OH |
| 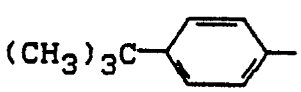 | -O-CH$_2$- | -CH-<br>OH | -S-CH$_2$- | -CH-<br>OH |

| Ar | X | Y | X | Y |
|---|---|---|---|---|
| 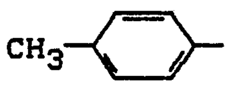 | $-O-CH_2-$ | $-CH(OH)-$ | $-S-CH_2-$ | $-CH(OH)-$ |
| 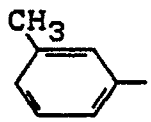 | $-O-CH_2-$ | $-CH(OH)-$ | $-S-CH_2-$ | $-CH(OH)-$ |
| 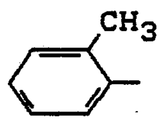 | $-O-CH_2-$ | $-CH(OH)-$ | $-S-CH_2-$ | $-CH(OH)-$ |
| 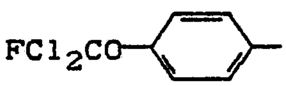 | $-O-CH_2-$ | $-CH(OH)-$ | $-S-CH_2-$ | $-CH(OH)-$ |
| 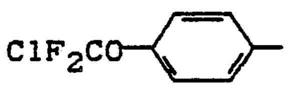 | $-O-CH_2-$ | $-C(=O)-$ | $-O-CH_2-$ | $-CH(OH)-$ |
| 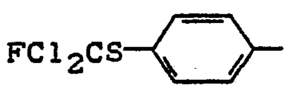 | $-O-CH_2-$ | $-C(=O)-$ | $-O-CH_2-$ | $-CH(OH)-$ |
| 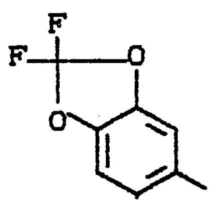 | $-O-CH_2-$ | $-C(=O)-$ | $-O-CH_2-$ | $-CH(OH)-$ |
| 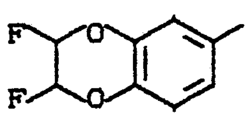 | $-O-CH_2-$ | $-C(=O)-$ | $-O-CH_2-$ | $-CH(OH)-$ |

| Ar | X | Y | X | Y |
|---|---|---|---|---|
| 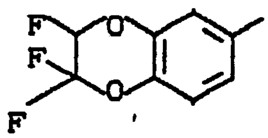 | $-O-CH_2-$ | 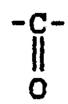 | $-O-CH_2-$ | 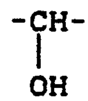 |
| 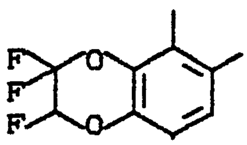 | $-O-CH_2-$ | 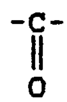 | $-O-CH_2-$ | 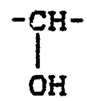 |
| 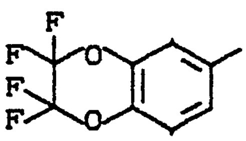 | $-O-CH_2-$ | 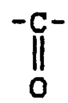 | $-O-CH_2-$ | 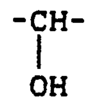 |
| 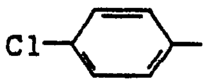 | $-O-CH_2-CH_2-$ | 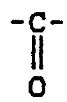 | $-O-CH_2-CH_2-$ | 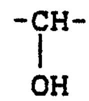 |
| 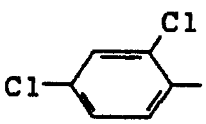 | $-O-CH_2-CH_2-$ | 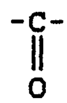 | $-O-CH_2-CH_2-$ | 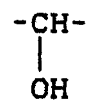 |
| 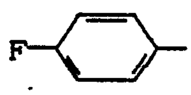 | $-O-CH_2-CH_2-$ | 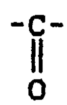 | $-O-CH_2-CH_2-$ | 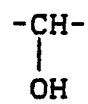 |
| 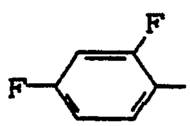 | $-O-CH_2-CH_2-$ | 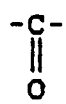 | $-O-CH_2-CH_2-$ | 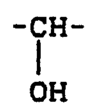 |
| 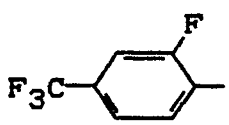 | $-O-CH_2-CH_2-$ | 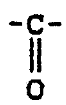 | $-O-CH_2-CH_2-$ | 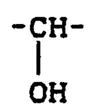 |

| Ar | X | Y | X | Y |
|---|---|---|---|---|
| 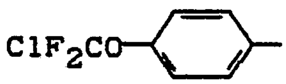 | -S-CH$_2$- | -C-<br>‖<br>O | -S-CH$_2$- | -CH-<br>│<br>OH |
| 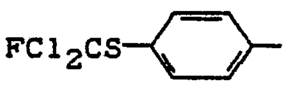 | -S-CH$_2$- | -C-<br>‖<br>O | -S-CH$_2$- | -CH-<br>│<br>OH |
| 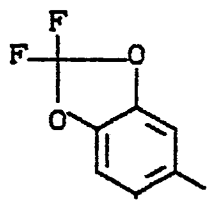 | -S-CH$_2$- | -C-<br>‖<br>O | -S-CH$_2$- | -CH-<br>│<br>OH |
| 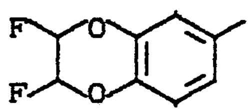 | -S-CH$_2$- | -C-<br>‖<br>O | -S-CH$_2$- | -CH-<br>│<br>OH |
| 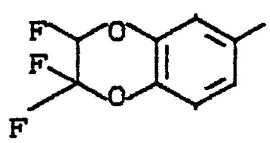 | -S-CH$_2$- | -C-<br>‖<br>O | -S-CH$_2$- | -CH-<br>│<br>OH |
| 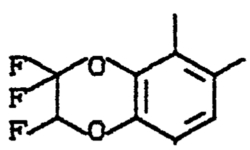 | -S-CH$_2$- | -C-<br>‖<br>O | -S-CH$_2$- | -CH-<br>│<br>OH |
| 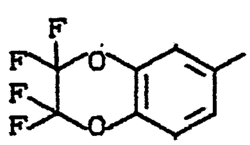 | -S-CH$_2$- | -C-<br>‖<br>O | -S-CH$_2$- | -CH-<br>│<br>OH |
| 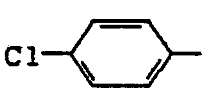 | -S-CH$_2$-CH$_2$- | -C-<br>‖<br>O | -S-CH$_2$-CH$_2$- | -CH-<br>│<br>OH |

| Ar | X | Y | X | Y |
|---|---|---|---|---|
| 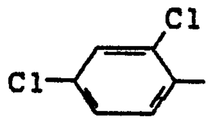 | $-S-CH_2-CH_2-$ | $-\underset{\underset{O}{\|}}{C}-$ | $-S-CH_2-CH_2-$ | $-\underset{\underset{OH}{\mid}}{CH}-$ |
| 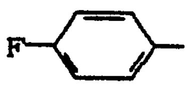 | $-S-CH_2-CH_2-$ | $-\underset{\underset{O}{\|}}{C}-$ | $-S-CH_2-CH_2-$ | $-\underset{\underset{OH}{\mid}}{CH}-$ |
| 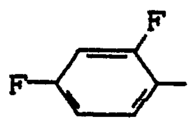 | $-S-CH_2-CH_2-$ | $-\underset{\underset{O}{\|}}{C}-$ | $-S-CH_2-CH_2-$ | $-\underset{\underset{OH}{\mid}}{CH}-$ |
| 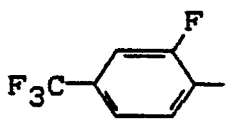 | $-S-CH_2-CH_2-$ | $-\underset{\underset{O}{\|}}{C}-$ | $-S-CH_2-CH_2-$ | $-\underset{\underset{OH}{\mid}}{CH}-$ |
| 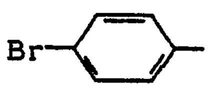 | $-O-CH_2-CH_2-$ | $-\underset{\underset{O}{\|}}{C}-$ | $-O-CH_2-CH_2-$ | $-\underset{\underset{OH}{\mid}}{CH}-$ |
| 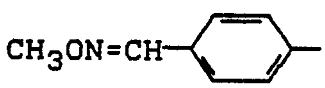 | $-O-CH_2-CH_2-$ | $-\underset{\underset{O}{\|}}{C}-$ | $-O-CH_2-CH_2-$ | $-\underset{\underset{OH}{\mid}}{CH}-$ |
| 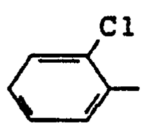 | $-O-CH_2-CH_2-$ | $-\underset{\underset{O}{\|}}{C}-$ | $-O-CH_2-CH_2-$ | $-\underset{\underset{OH}{\mid}}{CH}-$ |
| 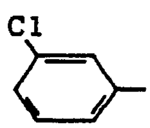 | $-O-CH_2-CH_2-$ | $-\underset{\underset{O}{\|}}{C}-$ | $-O-CH_2-CH_2-$ | $-\underset{\underset{OH}{\mid}}{CH}-$ |

| Ar | X | Y | X | Y |
|---|---|---|---|---|
| 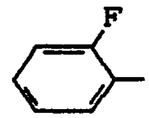 | $-O-CH_2-CH_2-$ | $-\underset{\underset{O}{\|}}{C}-$ | $-O-CH_2-CH_2-$ | $-\underset{\underset{OH}{\mid}}{CH}-$ |
| 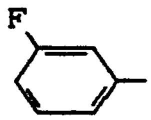 | $-O-CH_2-CH_2-$ | $-\underset{\underset{O}{\|}}{C}-$ | $-O-CH_2-CH_2-$ | $-\underset{\underset{OH}{\mid}}{CH}-$ |
| 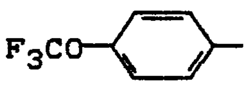 | $-O-CH_2-CH_2-$ | $-\underset{\underset{O}{\|}}{C}-$ | $-O-CH_2-CH_2-$ | $-\underset{\underset{OH}{\mid}}{CH}-$ |
| 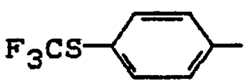 | $-O-CH_2-CH_2-$ | $-\underset{\underset{O}{\|}}{C}-$ | $-O-CH_2-CH_2-$ | $-\underset{\underset{OH}{\mid}}{CH}-$ |
| 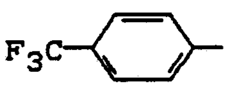 | $-O-CH_2-CH_2-$ | $-\underset{\underset{O}{\|}}{C}-$ | $-O-CH_2-CH_2-$ | $-\underset{\underset{OH}{\mid}}{CH}-$ |
| 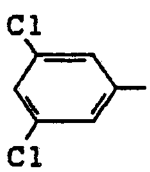 | $-O-CH_2-CH_2-$ | $-\underset{\underset{O}{\|}}{C}-$ | $-O-CH_2-CH_2-$ | $-\underset{\underset{OH}{\mid}}{CH}-$ |
| 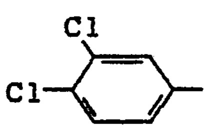 | $-O-CH_2-CH_2-$ | $-\underset{\underset{O}{\|}}{C}-$ | $-O-CH_2-CH_2-$ | $-\underset{\underset{OH}{\mid}}{CH}-$ |
| 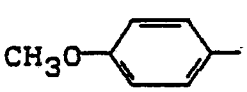 | $-O-CH_2-CH_2-$ | $-\underset{\underset{O}{\|}}{C}-$ | $-O-CH_2-CH_2-$ | $-\underset{\underset{OH}{\mid}}{CH}-$ |

| Ar | X | Y | X | Y |
|---|---|---|---|---|
| 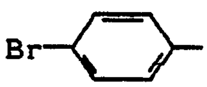 | $-S-CH_2-CH_2-$ | -C-<br>‖<br>O | $-S-CH_2-CH_2-$ | -CH-<br>\|<br>OH |
| 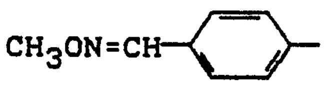 | $-S-CH_2-CH_2-$ | -C-<br>‖<br>O | $-S-CH_2-CH_2-$ | -CH-<br>\|<br>OH |
| 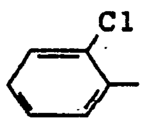 | $-S-CH_2-CH_2-$ | -C-<br>‖<br>O | $-S-CH_2-CH_2-$ | -CH-<br>\|<br>OH |
| 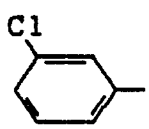 | $-S-CH_2-CH_2-$ | -C-<br>‖<br>O | $-S-CH_2-CH_2-$ | -CH-<br>\|<br>OH |
| 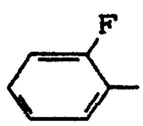 | $-S-CH_2-CH_2-$ | -C-<br>‖<br>O | $-S-CH_2-CH_2-$ | -CH-<br>\|<br>OH |
| 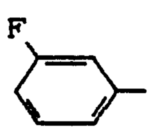 | $-S-CH_2-CH_2-$ | -C-<br>‖<br>O | $-S-CH_2-CH_2-$ | -CH-<br>\|<br>OH |
| 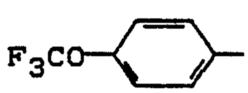 | $-S-CH_2-CH_2-$ | -C-<br>‖<br>O | $-S-CH_2-CH_2-$ | -CH-<br>\|<br>OH |
| 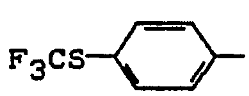 | $-S-CH_2-CH_2-$ | -C-<br>‖<br>O | $-S-CH_2-CH_2-$ | -CH-<br>\|<br>OH |

| Ar | X | Y | X | Y |
|---|---|---|---|---|
| 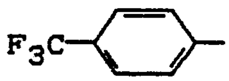 | $-S-CH_2-CH_2-$ | $-\underset{\underset{O}{\|}}{C}-$ | $-S-CH_2-CH_2-$ | $-\underset{\underset{OH}{\|}}{CH}-$ |
| 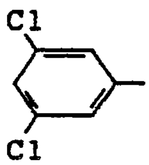 | $-S-CH_2-CH_2-$ | $-\underset{\underset{O}{\|}}{C}-$ | $-S-CH_2-CH_2-$ | $-\underset{\underset{OH}{\|}}{CH}-$ |
| 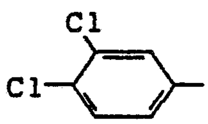 | $-S-CH_2-CH_2-$ | $-\underset{\underset{O}{\|}}{C}-$ | $-S-CH_2-CH_2-$ | $-\underset{\underset{OH}{\|}}{CH}-$ |
| 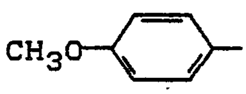 | $-S-CH_2-CH_2-$ | $-\underset{\underset{O}{\|}}{C}-$ | $-S-CH_2-CH_2-$ | $-\underset{\underset{OH}{\|}}{CH}-$ |
| 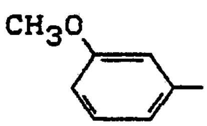 | $-O-CH_2-CH_2-$ | $-\underset{\underset{O}{\|}}{C}-$ | $-O-CH_2-CH_2-$ | $-\underset{\underset{OH}{\|}}{CH}-$ |
| 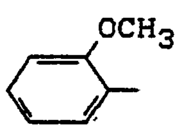 | $-O-CH_2-CH_2-$ | $-\underset{\underset{O}{\|}}{C}-$ | $-O-CH_2-CH_2-$ | $-\underset{\underset{OH}{\|}}{CH}-$ |
| 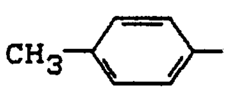 | $-O-CH_2-CH_2-$ | $-\underset{\underset{O}{\|}}{C}-$ | $-O-CH_2-CH_2-$ | $-\underset{\underset{OH}{\|}}{CH}-$ |
| 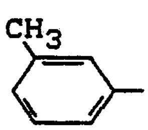 | $-O-CH_2-CH_2-$ | $-\underset{\underset{O}{\|}}{C}-$ | $-O-CH_2-CH_2-$ | $-\underset{\underset{OH}{\|}}{CH}-$ |

| Ar | X | Y | X | Y |
|---|---|---|---|---|
| 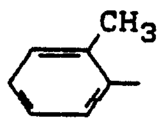 | -O-CH$_2$-CH$_2$- | -C-<br>‖<br>O | -O-CH$_2$-CH$_2$- | -CH-<br>\|<br>OH |
| 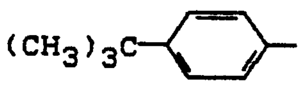 | -O-CH$_2$-CH$_2$- | -C-<br>‖<br>O | -O-CH$_2$-CH$_2$- | -CH-<br>\|<br>OH |
| 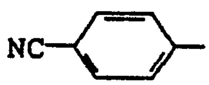 | -O-CH$_2$-CH$_2$- | -C-<br>‖<br>O | -O-CH$_2$-CH$_2$- | -CH-<br>\|<br>OH |
| 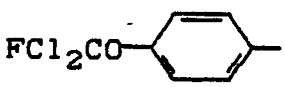 | -O-CH$_2$-CH$_2$- | -C-<br>‖<br>O | -O-CH$_2$-CH$_2$- | -CH-<br>\|<br>OH |
| 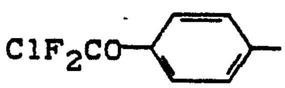 | -O-CH$_2$-CH$_2$- | -C-<br>‖<br>O | -O-CH$_2$-CH$_2$- | -CH-<br>\|<br>OH |
| 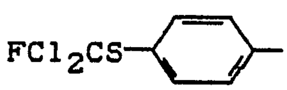 | -O-CH$_2$-CH$_2$- | -C-<br>‖<br>O | -O-CH$_2$-CH$_2$- | -CH-<br>\|<br>OH |
| 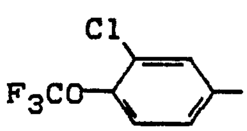 | -O-CH$_2$-CH$_2$- | -C-<br>‖<br>O | -O-CH$_2$-CH$_2$- | -CH-<br>\|<br>OH |
| 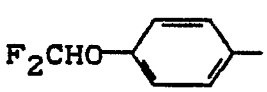 | -O-CH$_2$-CH$_2$- | -C-<br>‖<br>O | -O-CH$_2$-CH$_2$- | -CH-<br>\|<br>OH |

| Ar | X | Y | X | Y |
|---|---|---|---|---|
| 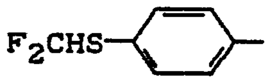 | $-O-CH_2-CH_2-$ | $-\underset{\underset{O}{\|}}{C}-$ | $-O-CH_2-CH_2-$ | $-\underset{\underset{OH}{\|}}{CH}-$ |
| 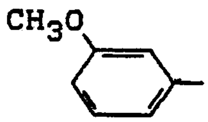 | $-S-CH_2-CH_2-$ | $-\underset{\underset{O}{\|}}{C}-$ | $-S-CH_2-CH_2-$ | $-\underset{\underset{OH}{\|}}{CH}-$ |
| 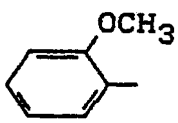 | $-S-CH_2-CH_2-$ | $-\underset{\underset{O}{\|}}{C}-$ | $-S-CH_2-CH_2-$ | $-\underset{\underset{OH}{\|}}{CH}-$ |
| 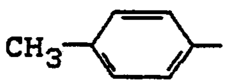 | $-S-CH_2-CH_2-$ | $-\underset{\underset{O}{\|}}{C}-$ | $-S-CH_2-CH_2-$ | $-\underset{\underset{OH}{\|}}{CH}-$ |
| 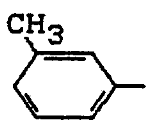 | $-S-CH_2-CH_2-$ | $-\underset{\underset{O}{\|}}{C}-$ | $-S-CH_2-CH_2-$ | $-\underset{\underset{OH}{\|}}{CH}-$ |
| 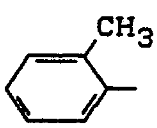 | $-S-CH_2-CH_2-$ | $-\underset{\underset{O}{\|}}{C}-$ | $-S-CH_2-CH_2-$ | $-\underset{\underset{OH}{\|}}{CH}-$ |
| 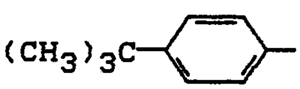 | $-S-CH_2-CH_2-$ | $-\underset{\underset{O}{\|}}{C}-$ | $-S-CH_2-CH_2-$ | $-\underset{\underset{OH}{\|}}{CH}-$ |
| 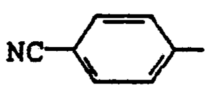 | $-S-CH_2-CH_2-$ | $-\underset{\underset{O}{\|}}{C}-$ | $-S-CH_2-CH_2-$ | $-\underset{\underset{OH}{\|}}{CH}-$ |

| Ar | X | Y | X | Y |
|---|---|---|---|---|
| 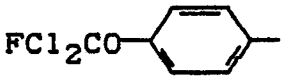 | $-S-CH_2-CH_2-$ | $-\underset{\underset{O}{\|}}{C}-$ | $-S-CH_2-CH_2-$ | $-\underset{\underset{OH}{\mid}}{CH}-$ |
| 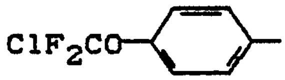 | $-S-CH_2-CH_2-$ | $-\underset{\underset{O}{\|}}{C}-$ | $-S-CH_2-CH_2-$ | $-\underset{\underset{OH}{\mid}}{CH}-$ |
| 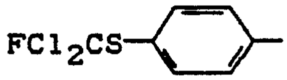 | $-S-CH_2-CH_2-$ | $-\underset{\underset{O}{\|}}{C}-$ | $-S-CH_2-CH_2-$ | $-\underset{\underset{OH}{\mid}}{CH}-$ |
| 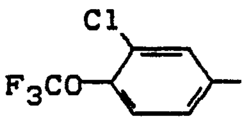 | $-S-CH_2-CH_2-$ | $-\underset{\underset{O}{\|}}{C}-$ | $-S-CH_2-CH_2-$ | $-\underset{\underset{OH}{\mid}}{CH}-$ |
| 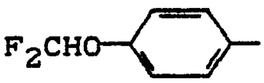 | $-S-CH_2-CH_2-$ | $-\underset{\underset{O}{\|}}{C}-$ | $-S-CH_2-CH_2-$ | $-\underset{\underset{OH}{\mid}}{CH}-$ |
| 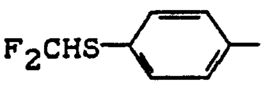 | $-S-CH_2-CH_2-$ | $-\underset{\underset{O}{\|}}{C}-$ | $-S-CH_2-CH_2-$ | $-\underset{\underset{OH}{\mid}}{CH}-$ |

Die erfindungsgemäß verwendbaren substituierten Pyrimidine der Formel (I) sind teilweise bekannt (vgl. DE-OS 34 31 689). Noch nicht bekannt sind Verbindungen der Formel (IA),

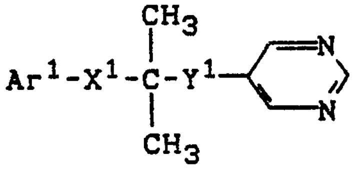

(IA)

in welcher

$Ar^1$ für gegebenenfalls substituiertes Phenyl steht,

$X^1$ für Sauerstoff, Schwefel, Sulfinyl, Sulfonyl oder für eine der Gruppen $-CH_2-$; $-O-CH_2-$; $-CH_2-O-$; $-O-CH_2-CH_2-$; $-S(O)_n-CH_2-$; $-CH_2-S(O)_n-$oder $-S(O)_n-CH_2-CH_2-$steht und

$Y^1$ für eine der Gruppen

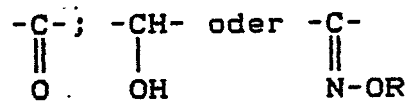

steht, wobei

R für Wasserstoff, Alkyl, Alkenyl, Alkinyl oder für gegebenenfalls substituiertes Aralkyl steht und

n für eine Zahl 0,1 oder 2 steht,

ausgenommen die Verbindungen 2,2-Dimethyl-3-(4-fluorphenyl)-1-(pyrimidin-5-yl)-propan-1-on, 2,2-Dimethyl-3-(3-chlorphenoxy)-1-(pyrimidin-5-yl)-propan-1-on, 2,2-Dimethyl-2-(4-chlorphenoxy)-1-(pyrimidin-5-yl)-ethan-1-on und 2,2-Dimethyl-3-(4-chlorphenylthio)-1-(pyrimidin-5-yl)-propan-1-on.

Die erfindungsgemäßen substituierten Pyrimidine sind durch die Formel (IA) allgemein definiert. Bevorzugt sind Verbindungen der Formel (IA), bei welchen

$Ar^1$ für gegebenenfalls einfach bis mehrfach, gleich oder verschieden substituiertes Phenyl steht, wobei als Substituenten infrage kommen: Halogen, Cyano, Nitro sowie jeweils geradkettiges oder verzweigtes Alkyl, Alkoxy, Alkylthio, Alkylsulfinyl oder Alkylsulfonyl mit jeweils 1 bis 4 Kohlenstoffatomen, jeweils geradkettiges oder verzweigte Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen, gegebenenfalls einfach bis mehrfach, gleich oder verschieden durch Halogen substituiertes, zweifach verknüpftes Dioxyalkylen oder jeweils gegebenenfalls einfach bis mehrfach, gleich oder verschieden durch Halogen substituiertes Phenyl oder Phenoxy,

$X^1$ für Sauerstoff, Schwefel, Sulfinyl, Sulfonyl oder für eine der Gruppen $-CH_2-$; $-O-CH_2-$; $-CH_2-O-$; $-O-CH_2-CH_2-$; $-S(O)_n-CH_2-S(O)_n-$oder $-S(O)_n-CH_2-CH_2-$steht und

$Y^1$ für eine der Gruppen

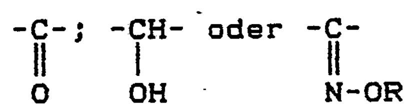

steht, wobei

R für Wasserstoff oder für jeweils geradkettiges oder verzweigtes Alkyl mit 1 bis 6 Kohlenstoffatomen, Alkenyl mit 3 bis 5 Kohlenstoffatomen oder Alkinyl mit 3 bis 5 Kohlenstoffatomen oder für gegebenenfalls einfach bis mehrfach, gleich oder verschieden substituiertes Benzyl oder Phenylethyl steht, wobei als Substituenten im Phenylteil jeweils die bei dem Rest $Ar^1$ genannten infrage kommen und

n für eine Zahl 0, 1 oder 2 steht,

ausgenommen die Verbindungen 2,2-Dimethyl-3-(4-fluorphenyl)-1-(pyrimidin-5-yl)-propan-1-on, 2,2-Dimethyl-3-(3-chlorphenoxy)-1-(pyrimidin-5-yl)-propan-1-on, 2,2-Dimethyl-2-(4-chlorphenoxy)-1-(pyrimidin-5-yl)-ethan-1-on und 2,2-Dimethyl-3-(4-chlorphenylthio)-1-(pyrimidin-5-yl)-propan-1-on.

Besonders bevorzugt sind die erfindungsgemäßen Verbindungen der Formel (IA), bei welchen

$Ar^1$ für gegebenenfalls ein-bis dreifach, gleich oder verschieden substituiertes Phenyl steht, wobei als Substituenten infrage kommen: Fluor, Chlor, Brom, Cyano, Nitro, Methyl, Ethyl, n-oder i-Propyl, n-, i-, s-oder t-Butyl, Methoxy, Ethoxy, n-oder i-Propoxy, Methylthio, Ethylthio, Trifluormethyl, Dichlorfluormethyl, Difluorchlormethyl, Difluorbrommethyl, Trichlormethyl, Fluormethyl, Difluormethyl, Trifluorethyl, Tetrafluorethyl, Trifluorchlorethyl, Pentafluorethyl, Trifluormethoxy, Difluormethoxy, Fluormethoxy, Dichlorfluormethoxy, Difluorchlormethoxy, Difluorbrommethoxy, Trichlormethoxy, Trifluorethoxy, Tetrafluorethoxy, Pentafluorethoxy, Trifluorchlorethoxy, Trifluordichlorethoxy, Difluortrichlorethoxy, Pentachlorethoxy, Trifluormethylthio, Difluormethylthio, Fluormethylthio, Difluorchlormethylthio, Dichlorfluormethylthio, Difluorethylthio, Difluorbrommethylthio, Trichlormethylthio, Trifluorethylthio, Tetrafluorethylthio, Pentafluorethylthio, Trifluorchlorethylthio, Trifluordichlorethylthio, Pentachlorethylthio, Methylsulfinyl, Trifluormethylsulfinyl, Dichlorfluormethylsulfinyl, Difluorchlormethylsulfinyl, Fluormethylsulfinyl, Difluormethylsulfinyl, Methylsulfonyl, Trifluormethylsulfonyl, Dichlorfluormethylsulfonyl, Difluorchlormethylsulfonyl, Fluormethylsulfonyl, Difluormethylsulfonyl, Methoxycarbonyl, Ethoxycarbonyl, Methoximinomethyl, Ethoximinomethyl, Methoximinoethyl, Ethoximinoethyl, Difluordioxymethylen, Tetrafluordioxyethylen, Trifluordioxyethylen, Difluordioxyethylen, Dioxymethylen, Dioxyethylen sowie jeweils gegebenenfalls ein-bis dreifach durch Fluor oder Chlor substituiertes Phenyl oder Phenoxy,
$X^1$ für Sauerstoff, Schwefel, Sulfinyl, Sulfonyl oder für eine der Gruppen $-CH_2-$; $-O-CH_2-$; $-CH_2-O-$; $-O-CH_2-CH_2-$; $-S(O)_n-CH_2-$; $-CH_2-S(O)_n-$oder $-S(O)_n-CH_2-CH_2-$steht und
$Y^1$ für eine der Gruppen

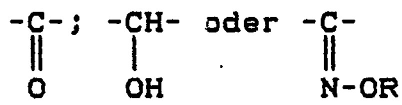
oder

steht, wobei
R für Wasserstoff, Methyl, Ethyl, n-oder i-Propyl, Allyl, Propargyl oder für gegebenenfalls ein-bis dreifach, gleich oder verschieden substituiertes Benzyl steht, wobei als Substituenten die bei dem Rest Ar genannten infrage kommen und
n für eine Zahl 0, 1 oder 2 steht,
ausgenommen die Verbindungen 2,2-Dimethyl-3-(4-fluorphenyl)-1-(pyrimidin-5-yl)-propan-1-on, 2,2-Dimethyl-3-(3-chlorphenoxy)-1-(pyrimidin-5-yl)-propan-1-on, 2,2-Dimethyl-2-(4-chlorphenoxy)-1-(pyrimidin-5-yl)-ethan-1-on und 2,2-Dimethyl-3-(4-chlorphenylthio)-1-(pyrimidin-5-yl)-propan-1-on.

Man erhält die bekannten Verbindungen ebenso wie die neuen Verbindungen nach Analogieverfahren.

So erhält man die noch nicht bekannten substituierten Pyrimidine der Formel (IA),

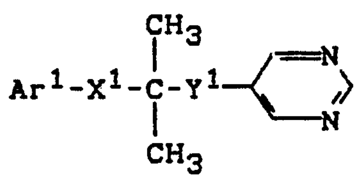
(IA)

in welcher
$Ar^1$ für gegebenenfalls substituiertes Phenyl steht,
$X^1$ für Sauerstoff, Schwefel, Sulfinyl, Sulfonyl oder für eine der Gruppen $-CH_2-$; $-O-CH_2-$; $-CH_2-O-$; $-O-CH_2-CH_2-$; $-S(O)_n-CH_2-$; $-CH_2-S(O)_n-$oder $-S(O)_n-CH_2-CH_2-$steht und
$Y^1$ für eine der Gruppen

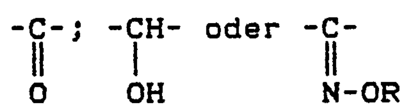
oder

steht, wobei
R für Wasserstoff, Alkyl, Alkenyl, Alkinyl oder für gegebenenfalls subsituiertes Aralkyl steht und
n für eine Zahl 0, 1 oder 2 steht,
ausgenommen die Verbindungen 2,2-Dimethyl-3-(4-fluorphenyl)-1-(pyrimidin-5-yl)-propan-1-on, 2,2-Dimethyl-3-(3-chlorphenoxy)-1-(pyrimidin-5-yl)-propan-1-on, 1,1-Dimethyl-2-(4-chlorphenoxy)-1-(pyrimidin-5-yl)-ethan-1-on und 2,2-Dimethyl-3-(4-chlorphenylthio)-1-(pyrimidin-5-yl)-propan-1-on,
sowie deren Säureadditionssalze und Metallsalzkomplexe in Analogie zu bekannten Methoden nach den im Folgenden aufgeführten Verfahren:

(a) Man erhält substituierte Pyrimidine der Formel (Ia)

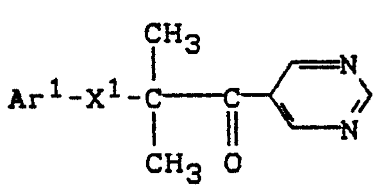
(Ia)

in welcher
$Ar^1$ und $X^1$ die oben angegebene Bedeutung haben,
wenn man Acylenole der Formel (II),

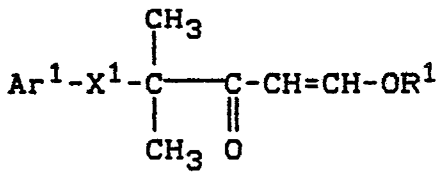 (II)

in welcher
$Ar^1$ und $X^1$ die oben angegebene Bedeutung haben und
$R^1$ für Wasserstoff oder ein Alkalimetallkation steht,
nacheinander mit Formamidin oder einem Säureadditionssalz des Formamidins, wie beispielsweise Formamidin Hydroacetat, und dann mit Formaldehyddimethylacetat gegebenenfalls in Gegenwart eines Verdünnungsmittels umsetzt;

(b) man erhält substituierte Pyrimidine der Formel (Ib),

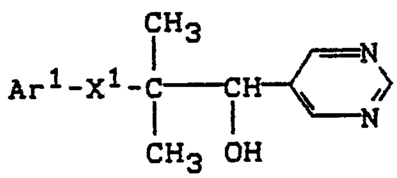 (Ib)

in welcher
$Ar^1$ und $X^1$ die oben angegebene Bedeutung haben,
wenn man die nach Verfahren (a) oder (d) erhältlichen substituierten Pyrimidine der Formel (Ia),

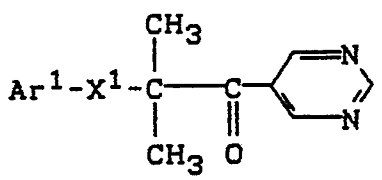 (Ia)

in welcher
$Ar^1$ und $X^1$ die oben angegebene Bedeutung haben,
mit einem Reduktionsmittel gegebenenfalls in Gegenwart eines Verdünnungsmittels umsetzt;

(c) man erhält substituierte Pyrimidine der Formel (Ic),

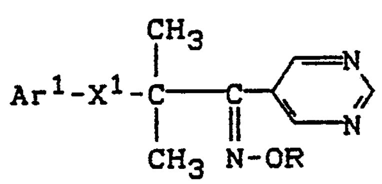 (Ic)

in welcher
$Ar^1$, $X^1$ und R die oben angegebene Bedeutung haben,
wenn man die nach Verfahren (a) oder (d) erhältlichen substituierten Pyrimidine der Formel (Ia),

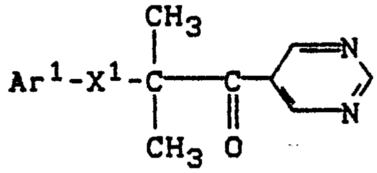

(Ia)

in welcher
$Ar^1$ und $X^1$ die oben angegebene Bedeutung haben,
mit Hydroxylaminen der Formel (III),
$R-O-NH_2$ (III)
in welcher
R die oben angegebene Bedeutung hat,
oder mit deren Säureadditionssalzen gegebenenfalls in Gegenwart eines Verdünnungsmittels und gegebenenfalls in Gegenwart eines Säurebindemittels umsetzt;

(d) man erhält substituierte Pyrimidine der Formel (Id),

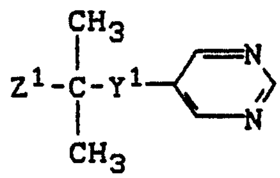

(Id)

in welcher
$Z^1$ für einen Rest $Ar^1$-$X^2$-oder für einen Rest $Ar^2$-$X^1$-steht, wobei
$Ar^1$, $X^1$ und $Y^1$ die oben angegebene Bedeutung haben,
$Ar^2$ für einen durch Alkylsulfinyl, Alkylsulfonyl, Halogenalkylsulfinyl oder Halogenalkylsulfonyl substituierten Phenylrest steht,
$X^2$ für Sulfinyl, Sulfonyl oder eine der Gruppen $-S(O)_m-CH_2-$; $-CH_2-S(O)_m-$oder $-S(O)_m-CH_2-CH_2-$steht und
m für eine Zahl 1 oder 2 steht,
wenn man die nach Verfahren (a), (b) oder (c) erhältlichen substituierten Pyrimidine der Formel (Ie),

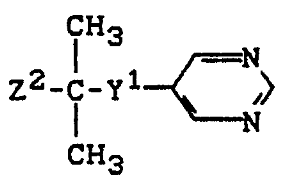

(Ie)

in welcher
$Z^2$ für einen Rest $Ar^1$-$X^3$-oder für einen Rest $Ar^3$-$X^1$-steht, wobei
$Ar^1$, $X^1$ und $Y^1$ die oben angegebene Bedeutung haben,
$Ar^3$ für einen durch Alkylthio oder Halogenalkylthio substituierten Phenylrest steht und
$X^3$ für Schwefel oder eine der Gruppen $-S-CH_2-$; $-CH_2-S-$oder $-S-CH_2-CH_2-$steht,
mit einem Oxidationsmittel, gegebenenfalls in Gegenwart eines Verdünnungsmittels und gegebenenfalls in Gegenwart eines Reaktionshilfsmittels umsetzt, und gegebenenfalls an die mit Hilfe der Herstellungsverfahren (a), (b), (c) oder (d) erhältlichen Verbindungen eine Säure oder ein Metallsalz addiert.

Verwendet man beispielsweise 4,4-Dimethyl-5-(4-trifluormethylthiophenyl)-1-penten-1-ol-3-on Natriumsalz als Ausgangsverbindung, so läßt sich der Reaktionsablauf des Herstellungsverfahrens (a) durch das folgende Formelschema darstellen:

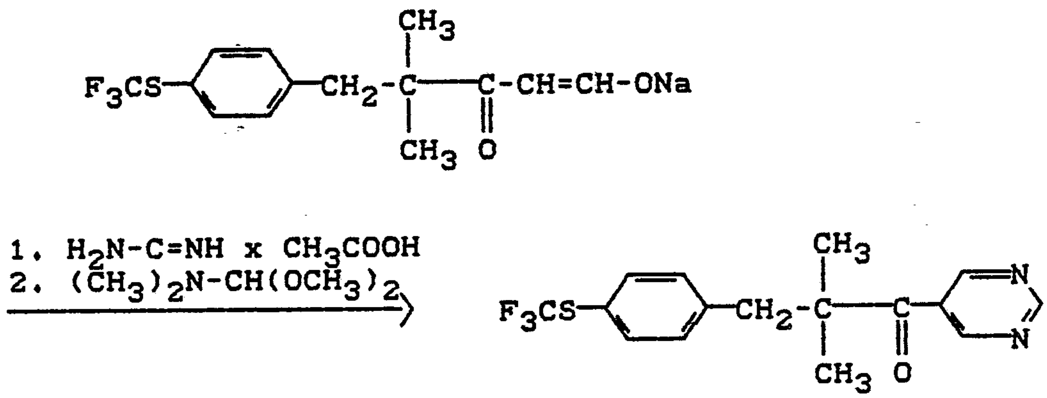


Verwendet man beispielsweise 1-(5-Pyrimidyl)-2,2-dimethyl-3-(4-trifluormethylthiophenyl)-propan-1-on als Ausgangsverbindung und Natriumborhydrid als Reduktionsmittel, so läßt sich der Reaktionsablauf des Herstellungsverfahren (b) durch das folgende Formelschema darstellen:

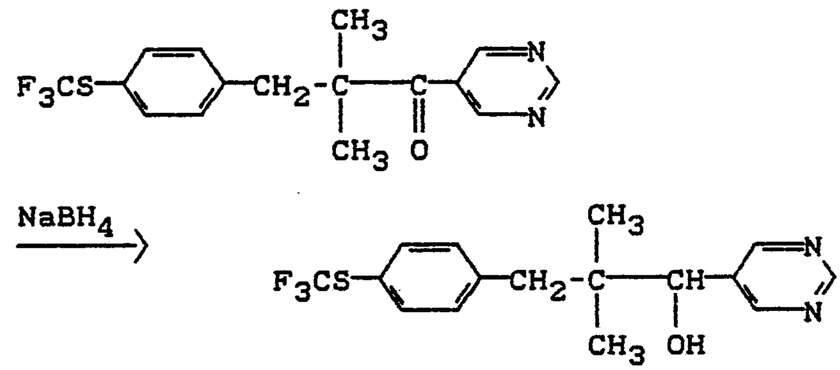


Verwendet man beispielsweise 1-(5-Pyrimidyl)-2,2-dimethyl-3-(4-trifluormethylphenyl)-propan-1-on und O-Methylhydroxylamin Hydrochlorid als Ausgangsstoffe, so läßt sich der Reaktionsablauf des Herstellungsverfahrens (c) durch das folgende Formelschema darstellen:

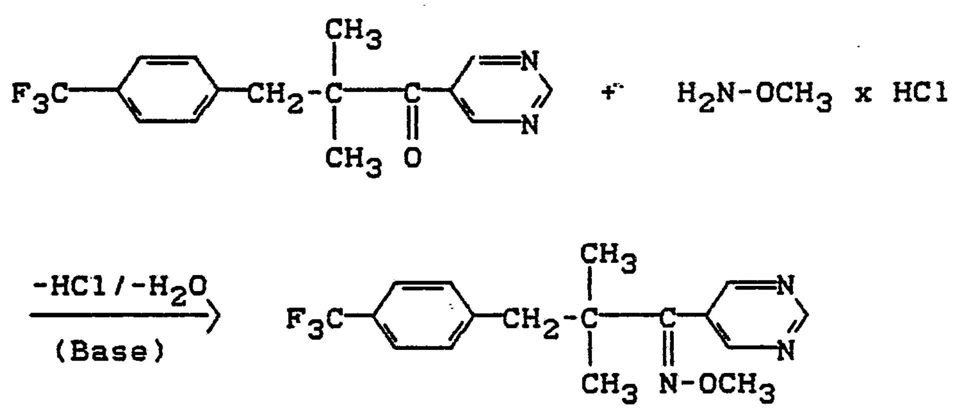


Verwendet man beispielsweise 1-(5-Pyrimidyl)-2,2-dimethyl-3-(4-trifluormethylthiophenyl)-propan-1-ol als Ausgangsverbindung und Wasserstoffperoxid als Oxidationsmittel, so läßt sich der Reaktionsablauf des Herstellungsverfahrens (d) durch das folgende Formelschema darstellen:

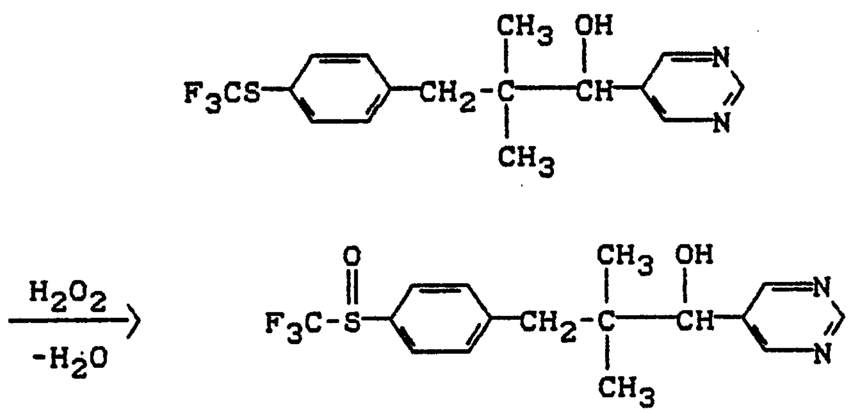


Die zur Durchführung des Herstellungsverfahrens (a) als Ausgangsstoffe benötigten Acylenole sind durch die Formel (II) allgemein definiert. In dieser Formel (II) stehen $Ar^1$ und $X^1$ vorzugsweise für diejenigen Reste, die bereits im Zusammenhang mit der Beschreibung der erfin dungsgemäß verwendbaren Stoffe der Formel (I) als bevorzugt für die Substituenten Ar und X genannt wurden.

Die Acylenole der Formel (II) sind teilweise bekannt oder erhältlich in Analogie zu bekannten Verfahren (vgl. z.B. DE-OS 34 31 689), beispielsweise, wenn man Ketone der Formel (IV),

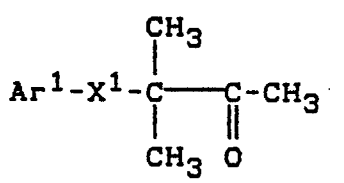
(IV)

in welcher
$Ar^1$ und $X^1$ die oben angegebene Bedeutung haben,
mit Ameisensäureethylester gegebenenfalls in Gegenwart einer Base, wie beispielsweise Natriumethylat oder Natriummethylat, und gegebenenfalls in Gegenwart eines Verdünnungsmittels, wie beispielsweise Diethylether, bei Temperaturen zwischen - 20 °C und + 60 °C umsetzt.

Die Ketone der Formel (IV) sind bekannt (vergl. z.B. DE-OS 32 10 725 oder DE-OS 30 48 266) oder lassen sich in Analogie zu bekannten Verfahren herstellen.

Die zur Durchführung der Herstellungsverfahren (b) und (c) als Ausgangsstoffe benötigten substituierten Pyrimidine sind durch die Formel (Ia) allgemein definiert. In dieser Formel (Ia) stehen $Ar^1$ und $X^1$ vorzugsweise für diejenigen Reste, die bereits im Zusammenhang mit der Beschreibung der erfindungsgemäß verwendbaren Stoffe der Formel (I) als bevorzugt für die Substituenten Ar und X genannt wurden, ausgenommen die Verbindungen 2,2-Dimethyl-3-(4-fluorphenyl)-1-(pyrimidin-5-yl)-propan-1-on, 2,2-Dimethyl-3-(3-chlorphenoxy)-1-(pyrimidin-5-yl)-propan-1-on, 2,2-Dimethyl-2-(4-chlorphenoxy)-1-(pyrimidin-5-yl)-ethan-1-on und 2,2-Dimethyl-3-(4-chlorphenylthio)-1-(pyrimidin-5-yl)-propan-1-on.

Die substituierten Pyrimidine der Formel (Ia) sind erfindungsgemäße Verbindungen und erhältlich mit Hilfe der Herstellungsverfahren (a) oder (d).

Die zur Durchführung des Herstellungsverfahrens (c) weiterhin als Ausgangsstoffe benötigten Hydroxylamine sind durch die Formel (III) allgemein definiert. In dieser Formel (III) steht R vorzugsweise für diejenigen Reste, die bereits im Zusammenhang mit der Beschreibung der erfindungsgemäß verwendbaren Stoffe der Formel (I) als bevorzugt für diesen Substituenten genannt wurden. Be vorzugte Säureadditionssalze der Hydroxylamine der Formel (III) sind Hydrohalogenide, wie insbesondere Hydrochloride oder Hydrobromide. Die Hydroxylamine der Formel (III) bzw. deren Säureaditionssalze wie Hydrochloride oder Hydrobromide sind allgemein bekannte Verbindungen.

Die zur Durchführung des Herstellungsverfahrens (d) als Ausgangsstoffe benötigten substituierten Pyrimidine sind durch die Formel (Ie) allgemein definiert. In dieser Formel (Ie) steht $X^1$ vorzugsweise für diejenigen Reste, die bereits im Zusammenhang mit der Beschreibung der erfindungsgemäß verwendbaren Stoffe der Formel (I) als bevorzugt für diesen Substituenten genannt wurden.

$Z^2$ steht für einen Rest $Ar^1$-$X^3$-oder für einen Rest $AR^3$-$X^1$-, wobei $Ar^1$ und $X^1$ vorzugsweise für diejenigen Reste stehen, die bereits im Zusammenhang mit der Beschreibung der erfindungsgemäß verwendbaren Stoffe der Formel (I) als bevorzugt für diese Substituenten genannt wurden, ausgenommen

die Verbindungen 2,2-Dimethyl-3-(4-fluorphenyl)-1-(pyrimidin-5-yl)-propan-1-on, 2,2-Dimethyl-3-(3-chlorphenoxy)-1-(pyrimidin-5-yl)-propan-1-on, 2,2-Dimethyl-2-(4-chlorphenoxy)-1-(pyrimidin-5-yl)ethan-1-on und 2,2-Dimethyl-3-(4-chlorphenylthio)-1-(pyrimidin-5-yl)-propan-1-on.

$Ar^3$ steht vorzugsweise für einen einfach bis mehrfach durch jeweils geradkettiges oder verzweigtes Alkylthio oder Halogenalkylthio mit jeweils 1 bis 4 Kohlenstoffatomen und gegebenenfalls 1 bis 9 gleichen oder verschiedenen Halogenatomen substituierten Phenylrest. Besonders bevorzugte Phenylsubstituenten sind dabei Methylthio, Ethylthio, Trifluormethylthio, Difluormethylthio, Fluormethylthio, Difluorchlormethylthio, Dichlorfluormethylthio, Difluorbrommethylthio, Trichlormethylthio, Trifluorethylthio, Tetrafluorethylthio, Pentafluorethylthio, Trifluorchlorethylthio, Trifluordichlorethylthio oder Pentachlorethylthio;

$X^3$ steht vorzugsweise für Schwefel oder eine der Gruppen $-CH_2-S-$; $-S-CH_2-$oder $-S-CH_2-CH_2-$.

Die substituierten Pyrimidine der Formel (Ie) sind erfindungsgemäße Verbindungen und erhältlich mit Hilfe der Herstellungsverfahren (a), (b) oder (c).

Als Verdünnungsmittel zur Durchführung des Herstellungsverfahrens (a) kommen inerte organische Lösungsmittel infrage. Vorzugsweise verwendet man aprotische Lösungsmittel wie beispielsweise Ether, insbesondere Diethylether, Diisopropylether, 1,2-Dimethoxyethan oder Tetrahydrofuran oder aromatische oder aliphatische Kohlenwasserstoffe, wie Benzol, Toluol oder Xylol, Petrolether, Hexan, Cyclohexan oder Octan.

Die Reaktionstemperaturen können bei der Durchführung des Herstellungsverfahrens (a) in einem größeren Bereich variiert werden. Im allgemeinen arbeitet man bei Temperaturen zwischen - 25 °C und + 100 °C, vorzugsweise bei Temperaturen zwischen 0 °C und 80 °C.

Zur Durchführung des Herstellungsverfahrens (a) setzt man pro Mol an Acylenol der Formel (II) im allgemeinen 1,0 bis 2,0 Mol, vorzugsweise 1,0 bis 1,5 Mol an Formamidin oder einem Säureadditionssalz des Formamidins, wie beispielsweise Formamidin Hydroacetat, und 1,0 bis 2,0 Mol, vorzugsweise 1,0 bis 1,5 Mol an Dimethylformamiddimethylacetal ein. Die Reaktionsdurchführung, Auafarbeitung und Isolierung der Reaktionsprodukte der Formel (Ia) erfolgt in Analogie zu bekannten Verfahren (vgl. DE-OS 34 31 689).

Als Reduktionsmittel zur Durchführung des Herstellungsverfahrens (b) kommen alle üblichen für derartige Ketonreduktionen gebräuchlichen Reduktionsmittel infrage. Vorzugsweise verwendet man komplexe Hydride, wie beispielsweise Lithiumaluminiumhydrid, Natriumborhydrid oder Natriumcyanoborhydrid oder Aluminium-organische Verbindungen, wie beispielsweise Aluminiumisopropylat.

Als Verdünnungsmittel zur Durchführung des Herstellungsverfahrens (b) kommen inerte organische Lösungsmittel infrage. Vorzugsweise verwendet man Kohlenwasserstoffe, wie Benzol oder Toluol; Ether, wie Diethylether oder Tetrahydrofuran oder Alkohole, wie Methanol, Ethanol, n-oder i-Propanol sowie n-oder i-Butanol.

Die Reaktionstemperaturen können bei der Durchführung des Herstellungsverfahrens (b) in einem größeren Bereich variiert werden. Im allgemeinen arbeitet man bei Temperaturen zwischen - 20 °C und + 120 °C, vorzugsweise bei Temperaturen zwischen 0 °C und 80 °C.

Zur Durchführung des Herstellungsverfahrens (b) setzt man pro Mol an substituiertem Pyrimidin der Formel (Ia) im allgemeinen 0,25 Mol bis 2 Mol, vorzugsweise äquimolare Mengen an Reduktionsmittel ein. Die Reaktionsdurchführung, Aufarbeitung und Isolierung der Reaktionsprodukte der Formel (Ib) erfolgt nach allgemein bekannten Verfahren.

Die mit Hilfe des Herstellungsverfahren (b) erhältlichen erfindungsgemäßen Carbinole der Formel (Ib) sind darüber hinaus auch interessante Zwischenprodukte zur Herstellung von weiteren Pflanzenschutzwirkstoffen. Sie können an der Hydroxylgruppe in allgemein bekannter Weise derivatisiert werden, beispielsweise mit Hilfe einer "Williamson Ethersynthese" in entsprechende Ether überführt werden oder durch Umsetzung mit Acylhalogeniden oder Carbamoylhalogeniden in entsprechende Ester oder Carbamate umgewandelt werden.

Als Verdünnungsmittel zur Durchführung des Herstellungsverfahrens (c) kommen polare organische Lösungsmittel oder deren Gemische mit Wasser infrage. Vorzugsweise verwendet man Alkohole, wie Methanol, Ethanol oder Propanol oder deren Gemische mit Wasser.

Das Herstellungsverfahren (c) kann gegebenenfalls in Gegenwart eines geeigneten Säurebindemittels durchgeführt werden.

Als solche kommen alle üblichen anorganischen oder organischen Basen in Frage. Hierzu gehören beispielsweise Alkalimetallhydroxide, wie Natriumhydroxid oder Kaliumhydroxid, Alkalimetallcarbonate oder -acetate, wie Natriumcarbonat, Kaliumcarbonat oder Natriumhydrogencarbonat oder Natriumacetat, sowie tertiäre Amine, wie Triethylamin, N,N-Dimethylanilin, Pyridin, N,N-Dimethylaminopyridin, Diazabicyclooctan (DABCO), Diazabicyclononen(DBN) oder Diazabicycloundecen(DBU).

Die Reaktionstemperaturen können bei der Durchführung des Herstellungsverfahrens (c) in einem größeren Bereich variiert werden. Im allgemeinen arbeitet man bei Temperaturen zwischen 0 °C und 120

°C, vorzugsweise bei Temperaturen zwischen 20 °C und 100 °C.

Zur Durchführung des Herstellungsverfahrens (c) setzt man pro Mol an substituiertem Pyrimidin der Formel (Ia) im allgemeinen 1,0 bis 2,0 Mol, vorzugsweise 1,0 bis 1,3 Mol an Hydroxylamin der Formel (III) vorzugsweise in Form des entsprechenden Hydrochlorides und 1,0 bis 2,0 Mol, vorzugsweise 1,0 bis 1,3 Mol an Säurebindemittel ein. Die Reaktionsdurchführung, Aufarbeitung und Isolierung der Reaktionsprodukte der Formel (Ie) erfolgt nach allgemein bekannten Verfahren.

Als Oxidationsmittel zur Durchführung des Herstellungsverfahrens (d) kommen alle üblicherweise für Schwefeloxidationen verwendbaren Oxidationsmittel infrage. Vorzugsweise verwendet man Wasserstoffperoxid oder organische Persäuren, wie beispielsweise Peressigsäure, 4-Nitroperbenzoesäure oder 3-Chlorperbenzoesäure oder auch anorganische Oxidationsmittel, wie Periodsäure, Kaliumpermanganat oder Chromsäure.

Als Verdünnungsmittel zur Durchführung des Herstellungsverfahrens (d) kommen in Abhängigkeit vom verwendeten Oxidationsmittel anorganische oder organische Lösungsmittel infrage. Vorzugsweise verwendet man Alkohole, wie Methanol oder Ethanol oder deren Gemische mit Wasser sowie reines Wasser; Säuren, wie beispielsweise Essigsäure, Acetanhydrid oder Propionsäure oder dipolaraprotische Lösungsmittel, wie Acetonitril, Aceton, Essigsäureethylester oder Dimethylformamid sowie auch gegebenenfalls halogenierte Kohlenwasserstoffe, wie Benzin, Benzol, Toluol, Hexan, Cyclohexan, Petrolether, Dichlormethan, Dichlorethan, Chloroform, Tetrachlorkohlenstoff oder Chlorbenzol.

Das Herstellungsverfahren (d) kann gegebenenfalls in Gegenwart eines Säurebindemittels durchgeführt werden.

Als solche kommen alle üblicherweise verwendbaren organischen und anorganischen Säurebindemittel in Frage. Vorzugsweise verwendet man Erdalkali-oder Alkalimetallhydroxide, -acetate oder -carbonate, wie beispielsweise Calciumhydroxid, Natriumhydroxid, Natriumacetate oder Natriumcarbonat.

Das Herstellungsverfahren (d) kann gegebenenfalls in Gegenwart eines geeigneten Katalysators durchgeführt werden. Als solche kommen alle üblicherweise für derartige Schwefeloxidationen verwendbaren Katalysatoren in Frage. Vorzugsweise verwendet man Schwermetallkatalysatoren; beispielhaft genannt sei in diesem Zusammenhang Ammoniummolybdat.

Die Reaktionstemperaturen können bei der Durchführung des Herstellungsverfahrens (d) in einem größeren Bereich variiert werden. Im allgemeinen arbeitet man bei Temperaturen zwischen - 30 °C und + 100 °C, vorzugsweise bei Temperaturen zwischen 0 °C und + 80 °C.

Zur Durchführung des Herstellungverfahren (d) setzt man pro Mol substituiertem Pyrimidin der Formel (Ie) im allgemeinen 0,8 bis 1,2 Mol, vorzugsweise äquimolare Mengen an Oxidationsmittel ein, wenn man die Oxidation des Schwefels auf der Sulfoxidstufe unterbrechen will. Für eine Oxidation bis zur Sulfonstufe setzt man pro Mol an substituiertem Pyrimidin der Formel (Ie) im allgemeinen 1,8 bis 3,0 Mol, vorzugsweise doppelt molare Mengen an Oxidationsmittel ein. Die Reaktionsdurchführung, Aufar beitung und Isolierung der Reaktionsprodukte der Formel (Id) erfolgt nach allgemein bekannten Verfahren.

Zur Herstellung von pflanzenverträglichen Säureadditionssalzen der Verbindungen der Formel (I) kommen vorzugsweise folgende Säuren infrage: Halogenwasserstoffsäuren, wie z.B. Chlorwasserstoffsäure und Bromwasserstoffsäure, insbesondere Chlorwasserstoffsäure, ferner Phosphorsäure, Salpetersäure, Schwefelsäure, mono-, bi-und trifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure, Milchsäure, sowie Sulfonsäuren, wie z.B. p-Toluolsulfonsäure und 1,5-Naphthalindisulfonsäure.

Die Säureadditionssalze der Verbindungen der Formel (I) können in einfacher Weise nach üblichen Salzbildungsmethoden, wie z.B. durch Lösen einer Verbindung der Formel (I) in einem geeigneten inerten Lösungsmittel und Hinzufügen der Säure, wie z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise Salze von Metallen der II. bis IV. Haupt-und der I. und II. sowie IV. bis VIII. Nebengruppe infrage, wobei Kupfer, Zink, Mangan, Magnesium, Zinn, Eisen und Nickel beispielhaft genannt seien.

Als Anionen von Salzen kommen solche in Betracht, die sich vorzugsweise von folgenden Säuren ableiten: Halogenwasserstoffsäure, wie z.B. Chlorwasserstoffsäure und Bromwasserstoffsäure, ferner Phosphorsäure, Salpetersäure und Schwefelsäure.

Die Metallsalzkomplexe von Verbindungen der Formel (I) können in einfacher Weise nach üblichen Verfahren erhalten werden, so z.B. durch Lösen des Metallsalzes in Alkohol, z.B. Ethanol und Hinzufügen zur Verbindung der Formel (I). Man kann Metallsalz-Komplexe in bekannter Weise, z.B. durch Abfiltrieren, isolieren und gegebenenfalls durch Umkristallisation reinigen.

Die erfindungsgemäß verwendbaren Wirkstoffe weisen eine starke Wirkung gegen Schädlinge auf und

können zur Bekämpfung von unerwünschten Schadorganismen praktisch eingesetzt werden. Die Wirkstoffe sind u.a. für den Gebrauch als Pflanzenschutzmittel, insbesondere als Fungizide und Bakterizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Bakterizide Mittel werden im Pflanzenschutz zur Bekämpfung von Pseudomonadaceae, Rhizobiaceae, Enterobacteriaceae, Corynebacteriaceae und Streptomycetaceae eingesetzt.

Beispielhaft aber nicht begrenzend seien einige Erreger von pilzlichen und bakteriellen Erkrankungen, die unter die oben aufgezählten Oberbegriffe fallen, genannt:

Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea (Konidienform: Drechslera, Syn: Helminthosporium);
Cochliobolus-Arten, wie beispielsweise Cochliobolus sativus (Konidienform: Drechslera, Syn: Helminthosporium);
Uromyces-Arten, wie beispielsweise Uromyces appendiculatus;
Puccinia-Arten, wie beispielsweise Puccinia recondità;
Tilletia-Arten, wie beispielsweise Tilletia caries;
Utilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia aryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz-und Saatgut, und des Bodens.

Dabei lassen sich die erfindungsgemäß verwendbaren Wirkstoffe mit besonders gutem Erfolg zur Bekämpfung von Getreidekrankheiten, wie beispielsweise gegen den Erreger der Braunspelzigkeit des Weizens (Leptosphaeria nodorum) oder gegen den Erreger der Netzfleckenkrankheit der Gerste (Pyrenophora teres) oder gegen den Erreger der Braunfleckenkrankheit im Getreide (Cochliobolus sativus), gegen Mehltau und Rostarten, sowie zur Bekämpfung von Krankheiten im Gemüse-und Obstbau, wie beispielsweise gegen den Erreger des Apfelschorfes (Venturia inaequalis) und gegen Botrytis-Arten sowie zur Bekämpfung von Reiskrankheiten, wie beispielsweise gegen den Erreger der Reisfleckenkrankheit (Pyricularia oryzae) einsetzen. Darüberhinaus zeigen die erfindungsgemäß verwendbaren Wirkstoffe auch gute bakterizide und pflanzenwuchsregulierende Eigenschaften.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Wirkstoff-imprägnierte Natur-und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt-und Warmnebel-Formulierungen.

Diese Formulierungen werden in bekannter Weise hergestellt, z.B. durch Vermischen der Wirkstoffe mit Streck mitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln

und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit so wie synthetische Granulate aus anaorganischen und organischen Mehlen sowie Granulate aus organischem Material wie Sägemehl, Kokosnußschalen, Maiskolben und Tabakstengel; als Emulgier und/oder - schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo-und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäß verwendbaren Wirkstoffe können in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

die Wirkstoffe konnen als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 % am Wirkungsort erforderlich.

<u>Herstellungsbeispiele</u>

Beispiel 1:

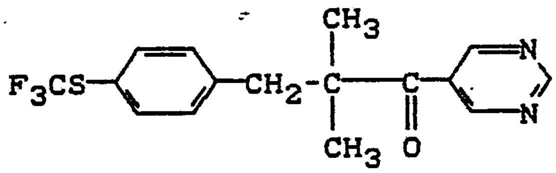

(Verfahren a)

97,0 g (0,298 Mol) 4,4-Dimethyl-5-(4-trifluormethylthiophenyl)-1-penten-1-ol-3-on Natriumsalz und 39,0 g (0,374 Mol) Formamidin Hydroacetat in 500 ml Tetrahydrofuran werden 30 Minuten bei Raumtemperatur gerührt und dann tropfenweise unter Rühren mit 48,5 g (0,406 Mol) Dimethylformamiddimethylacetal versetzt. Man rührt 6 Stunden bei 50 °C bis 60 °C, kühlt dann auf Raumtemperatur ab, saugt ab, engt das Filtrat im Vakuum ein und reinigt den Rückstand chromatographisch (Kieselgel; Diethylether).
Man erhält 18,5 g (18,3 % der Theorie) an 1-(5-Pyrimidyl)-2,2-dimethyl-3-(4-trifluormethylthiophenyl)propan-1-on als Öl.

$^1$H-NMR ($CDCl_3$/TMS) : $\delta$ = 1,75; 3,10; 8,89; 9,29 ppm.

Herstellung der Ausgangsverbindung

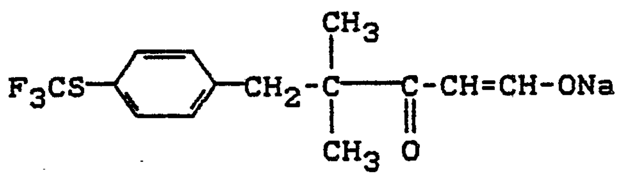

Zu 129,4 g (0,469 Mol) 3,3-Dimethyl-4-(4-trifluormethylthiophenyl)-butan-2-on und 25,3 g (0,469 Mol) Natriummethylat in 1 l Diethylether tropft man bei Raumtemperatur unter Rühren 34,7 g (0,469 Mol) Ameisensäureethylester und rührt nach beendeter Zugabe 4 Stunden bei Raumtemperatur. Zur Aufarbeitung extrahiert man mit 1 l Wasser, wäscht die vereinigten wässrigen Extrakte einmal mit Diethylether, säuert mit verdünnter wässriger Chlorwasserstoffsäure auf pH 1 - 2 an und extrahiert mit Dichlormethan. Die Dichlormethanphase wird über Natriumsulfat getrocknet und im Vakuum eingeengt. Man erhält 90,5 g 4,4-Dimethyl-5-(4-trifluormethylthiophenyl)-penten-1-ol-3-on, welches in 200 ml Methanol gelöst und mit 17,0 g (0,315 Mol) Natriummethylat versetzt wird. Einengen der Lösung im Vakuum ergibt 97,1 g (75 % der Theorie) an 4,4-Dimethyl-5-(4-trifluormethylthiophenyl)-1-penten-1-ol-3-on Natriumsalz als Harz, welches ohne Reinigung weiter umgesetzt wird.

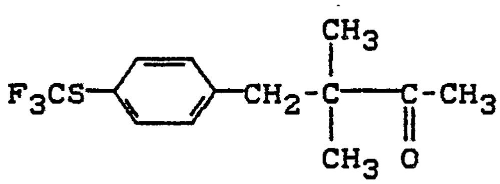

147,8 g (2,64 Mol) gepulvertes Kaliumhydroxid und 8,8 g (0,027 Mol) Tetrabutylammoniumbromid werden in 270 ml Toluol bei Raumtemperatur vorgelegt. Bei 20 °C bis 25 °C läßt man unter Kühlung eine Mischung aus 200 g (0,88 Mol) 4-Trifluormethylthio-benzylchlorid und 90,9 g (1,06 Mol) Methylisopropylketon zulaufen.
Nach Beendigung der Zugabe rührt man 8 Stunden bei 20 °C bis 25 °C nach.
Zur Aufarbeitung versetzt man mit 500 ml Wasser, trennt die Phasen, trocknet die organische Phase über Natriumsulfat und engt im Wasserstrahlvakuum ein. Der Rückstand wird fraktioniert destilliert.
Man erhält 115 g (47,3 % der Theorie) an 3,3-Dimethyl-4-(4-trifluormethylthiophenyl)-butan-2-on vom Siedepunkt Kp : 100 °C bei 0,8 mbar.

Beispiel 2:

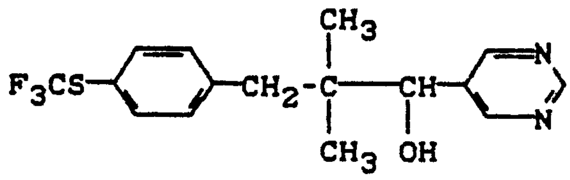

(Verfahren b)

Zu 10 g (0,0294 Mol) 1-(5-Pyrimidyl)-2,2-dimethyl-3-(4-trifluormethylthiophenyl)-propan-1-on in 70 ml Methanol gibt man 0,37 g (0,0098 Mol) Natriumborhydrid in 7 ml Wasser und rührt 30 Minuten bei Raumtemperatur. Zur Aufarbeitung engt man im Vakuum ein, nimmt den Rückstand in Dichlormethan auf, trocknet über Natriumsulfat und entfernt das Lösungsmittel im Vakuum.

Man erhält 10,05 g (100 % der Theorie) am 1-(5-Pyrimidyl)-2,2-dimethyl-3-(4-trifluormethylthiophenyl)-propan-1-ol vom Brechungsindex $n_D^{20}$ 1.5300.

Beispiel 3:

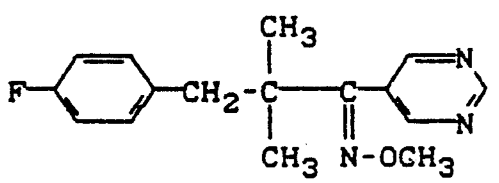

(Verfahren c)

8,0 g (0,031 Mol) 1-(5-Pyrimidinyl)-2,2-dimethyl-3-(4-fluorphenyl)-propan-1-on (vgl. DE-OS 34 31 689), 4,0 g (0,048 Mol) O-Methylhydroxylamin Hydrochlorid und 4,0 g (0,049 Mol) Natriumacetat werden in einem Gemisch aus 50 ml Methanol und 10 ml Wasser 15 Stunden bei Raumtemperatur gerührt. Zur Aufarbeitung wird der Ansatz mit 200 ml Wasser verdünnt, ausgefallenes Reaktionsprodukt abgesaugt und in Methylenchlorid gelöst. Die Methylenchloridlösung wird über Natriumsulfat getrocknet und im Vakuum eingeengt.

Man erhält 5,7 g (64,1 % der Theorie) an 1-Methoximino-1-(5-pyrimidinyl)-2,2-dimethyl-3-(4-fluorphenyl)-propan als Öl.

$^1$H-NMR ($CDCl_3$/Tetramethylsilan) : $\delta$ = 1.10 (s,6H); 2,86 (s,2H); 3,80 (s,3H) ppm.

Beispiel 4:

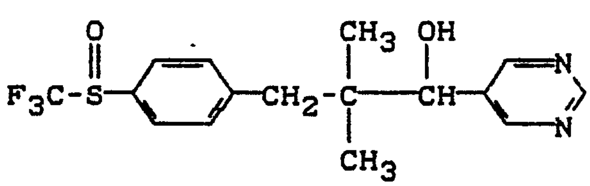

(Verfahren d)

Zu 2 g (0,0058 Mol) 1-(5-Pyrimidyl)-2,2-dimethyl-3-(4-trifluormethylthiophenyl)-propan-1-ol in 10 ml Eisessig gibt man 1,74 g (0,0174 Mol) 35prozentige wässrige Wasserstoffperoxidlösung, rührt 8 Stunden bei 50 °C, kühlt ab, verrührt mit 100 ml Wasser, extrahiert mit Dichlormethan, neutralisiert die Dichlormethanphase mit wässrigem Natriumhydrogencarbonat, wäscht mit Wasser, trocknet über Natriumsulfat, engt im Vakuum ein und reinigt den Rückstand chromatographisch (Kieselgel; Essigsäureethylester/Cyclohexan 3:1).

Man erhält 0,5 g (23 % der Theorie) an 1-(5-Pyrimidyl)-2,2-dimethyl-3-(4-trifluormethylsulfinylphenyl)-propan-1-ol vom Schmelzpunkt 122 °C.

In entsprechender Weise und gemäß den allgemeinen Angaben zur Herstellung erhält man die folgenden substituierten Pyrimidine der allgemeinen Formel (I):

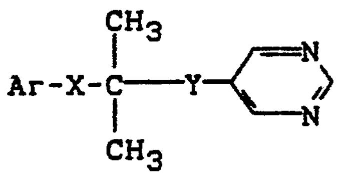

(I)

| Bsp. Nr. | Ar | X | Y | physikalische Eigenschaften |
|---|---|---|---|---|
| 5 | 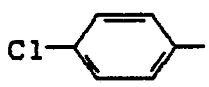 | O | -C-<br>‖<br>O | Fp 106 °C |
| 6 | 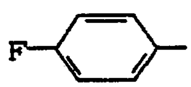 | $-CH_2-$ | -C-<br>‖<br>O | $n_D^{20}$ 1.5473 |
| 7 | 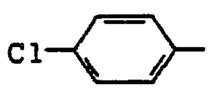 | O | -CH-<br>\|<br>OH | Fp 100 °C |
| 8 | 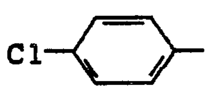 | $-S-CH_2-$ | -C-<br>‖<br>O | Fp 58 °C |
| 9 | 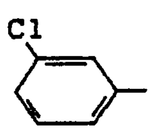 | $-O-CH_2-$ | -C-<br>‖<br>O | Fp 178 °C |
| 10 | 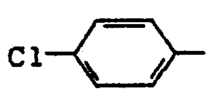 | $-CH_2-$ | -C-<br>‖<br>O | Fp 65 °C |

| Bsp. Nr. | Ar | X | Y | physikalische Eigenschaften |
|---|---|---|---|---|
| 11 | 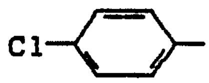 | $-CH_2-$ | -C-<br>‖<br>$N-OCH_3$ | Fp 102 °C |
| 12 | 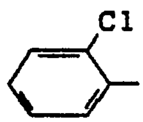 | $-CH_2-$ | -C-<br>‖<br>O | ¹H-NMR*): 1,37(6H)<br>3,25(2H)<br>8,96(2H)<br>9,27(1H) |
| 13 | 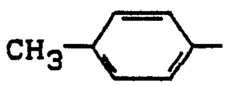 | $-CH_2-$ | -C-<br>‖<br>O | Fp 60 °C |
| 14 | 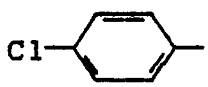 | $-CH_2-$ | -CH-<br>\|<br>OH | Fp 129 °C |
| 15 | 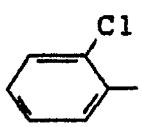 | $-CH_2-$ | -C-<br>‖<br>$N-OCH_3$ | Fp 74 °C |
| 16 | 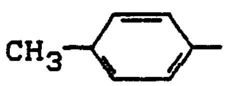 | $-CH_2-$ | -C-<br>‖<br>$N-OCH_3$ | Fp 74 °C |
| 17 | 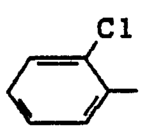 | $-CH_2-$ | -CH-<br>\|<br>OH | Fp 110 °C |

| Bsp. Nr. | Ar | X | Y | physikalische Eigenschaften |
|---|---|---|---|---|
| 18 | 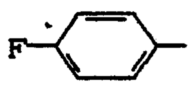 | $-CH_2-$ | 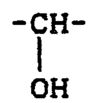 | Fp 115 °C |
| 19 | 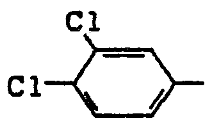 | $-CH_2-$ | 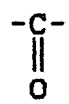 | Fp 105 °C |
| 20 | 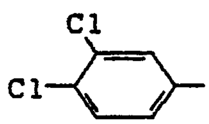 | $-CH_2-$ | 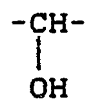 | Fp 107 °C |
| 21 | 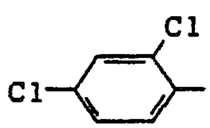 | $-CH_2-$ | 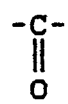 | Fp 99 °C |
| 22 | 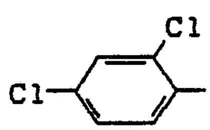 | $-O-CH_2-$ | 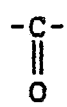 | Fp 44 °C |
| 23 | 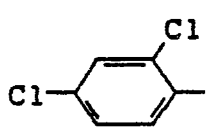 | $-O-CH_2-$ | 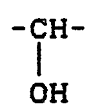 | Fp 84 °C |
| 24 | 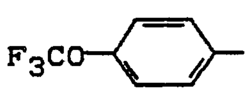 | $-CH_2-$ | 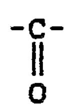 | $n_D^{20}$ 1.4994 |

| Bsp. Nr. | Ar | X | Y | physikalische Eigenschaften |
|---|---|---|---|---|
| 25 | 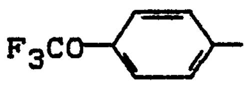 | $-CH_2-$ | 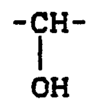 | $n_D^{20}$ 1.5009 |
| 26 | 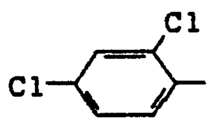 | $-CH_2-$ | 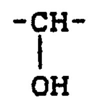 | Fp 105 °C |
| 27 | 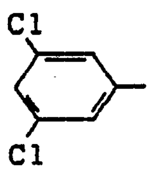 | O | 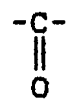 | Fp 129 °C |
| 28 | 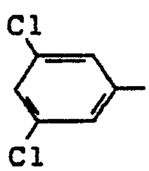 | O | 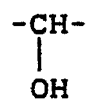 | Fp 102 °C |
| 29 | 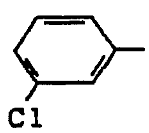 | $-O-CH_2-$ | 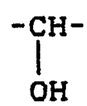 | Fp 124 °C |
| 30 | 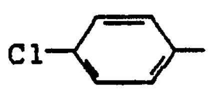 | $-S-CH_2-$ | 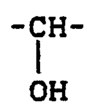 | Fp 141 °C |
| 31 | 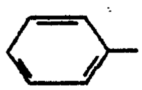 | $-S-CH_2-$ | 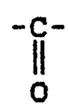 | Fp 89 °C |
| 32 | 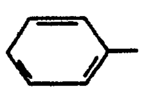 | $-O-CH_2-$ | 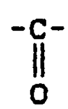 | $n_D^{20}$ 1.5492 |

| Bsp. Nr. | Ar | X | Y | physikalische Eigenschaften |
|---|---|---|---|---|
| 33 | 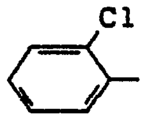 | $-O-CH_2-$ | 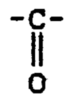 | $^1H$-NMR:*) 1,51(6H); 4,12(2H); 9,09(2H); 9,29(1H) |
| 34 | 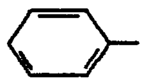 | $-S-CH_2-$ | 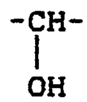 | Fp 68 °C |
| 35 | 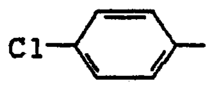 | $-O-CH_2-$ | 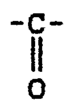 | Fp 112 °C |
| 36 | 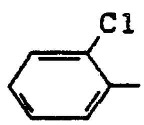 | $-O-CH_2-$ | 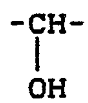 | Fp 90 °C |
| 37 | 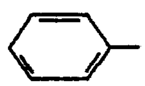 | $-O-CH_2-$ | 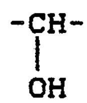 | Fp 74 °C |
| 38 | 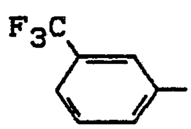 | $-O-CH_2-$ | 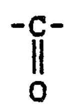 | $^1H$-NMR*) 1,51 (6H); 4,14 (2H); 9,02 (2H); 9,30 (1H) |
| 39 | 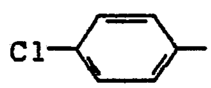 | $-O-CH_2-$ | 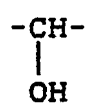 | Fp 109 °C |

| Bsp. Nr. | Ar | X | Y | physikalische Eigenschaften |
|---|---|---|---|---|
| 40 | 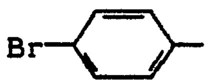 | $-S-CH_2-$ | 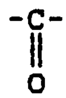 | Fp 96 °C |
| 41 | 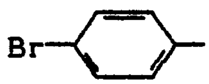 | $-S-CH_2-$ | 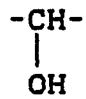 | Fp 150 °C |
| 42 | 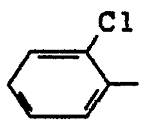 | $-S-CH_2-$ | 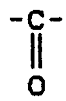 | Fp 59 °C |
| 43 | 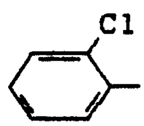 | $-S-CH_2-$ | 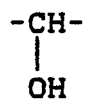 | Fp 118 °C |
| 44 | 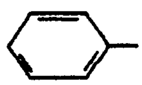 | $-S-CH_2-$ | 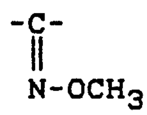 | $^1$H-NMR*): 1,30 (6H); 3,10 (2H); 3,81 (3H) |
| 45 | 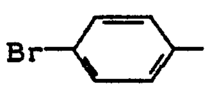 | $-S-CH_2-$ | 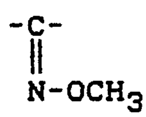 | $^1$H-NMR*): 1,29 (6H); 3,07 (2H); 3,80 (3H) |
| 46 | 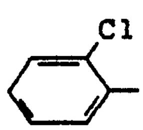 | $-S-CH_2-$ | 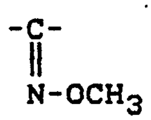 | $^1$H-NMR*): 1,29 (6H); 3,08 (2H); 3,79 (3H) |

| Bsp. Nr. | Ar | X | Y | physikalische Eigenschaften |
|---|---|---|---|---|
| 47 | 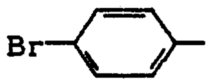 | $CH_2$ | 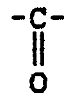 | Fp. 114° C |
| 48 | 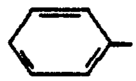 | $CH_2$ | 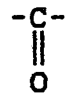 | $^1$H-NMR*): 3,04 (2H); 1,32 (6H) |
| 49 | 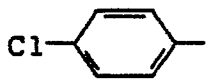 | S | 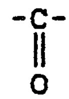 | FP. 70° C |
| 50 | 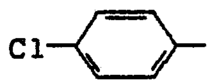 | S | 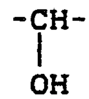 | $^1$H-NMR*): 4,40 (1H); 8,70 (2H); 9,15 (1H) |

*) Die $^1$H-NMR-Spektren wurden in $CDCl_3$ mit Tetramethylsilan (TMS) als innerem Standard aufgenommen. Angegeben ist die chemische Verschiebung als δ-Wert in ppm.

Anwendungsbeispiele

In den folgenden Anwendungsbeispielen wurden die nachstehend aufgeführten Verbindungen als Vergleichsubstanzen eingesetzt:

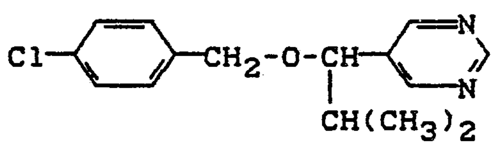 (A)

5-[1-(4-Chlorbenzyloxy)-2-methyl-prop-1-yl]-pyrimidin und

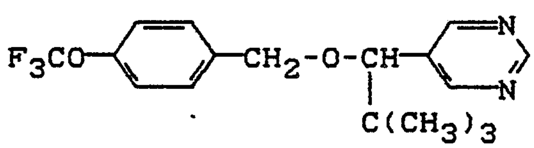 (B)

5-[1-(4-Trifluormethoxybenzyloxy)-2,2-dimethyl-prop-1-yl]-pyrimidin (beide bekannt aus DE-OS 31 05 374).

Beispiel A

Leptosphaeria nodorum-Test (Weizen)/protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Leptosphaeria nodorum besprüht. Die Pflanzen verbleiben 48 Stunden bei 20 °C und 100 % relativer Luftfeuchtigkeit in einer Inkubationskabine.

Die Pflanzen werden in einem Gewächshaus bei einer Temperatur von ca. 15 °C und einer relativen Luftfeuchtigkeit von ca. 80 % aufgestellt.

10 Tage nach der Inokulation erfolgt die Auswertung.

Eine deutliche Überlegenheit in der Wirksamkeit gegenüber dem Stand der Technik zeigen bei diesem Test z.B. die Verbindungen gemäß folgender Herstellungsbeispiele: 1, 2, 5, 8, 9, 10, 13, 14, 19, 20, 21, 24, 25.

## Tabelle A

Leptosphaeria nodorum-Test (Weizen)/protektiv

| Wirkstoff | Wirkstoffkonzentration in der Spritzbrühe in Gew.-% | Krankheitsbefall in % der unbehandelten Kontrolle |
|---|---|---|
| 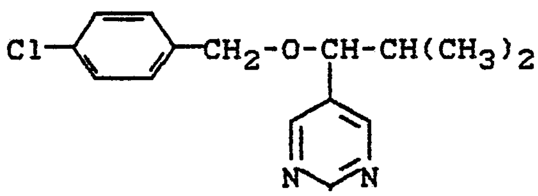 (bekannt) (A) | 0,025 | 75,0 |
| 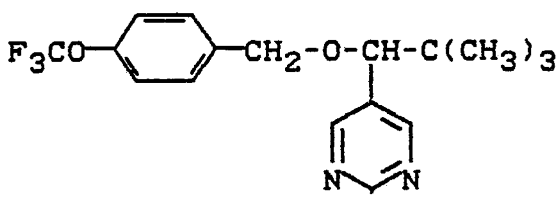 (bekannt) (B) | 0,025 | 100 |
| 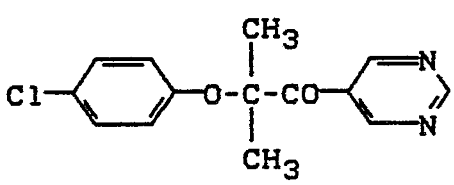 (5) | 0,025 | 25,0 |
| 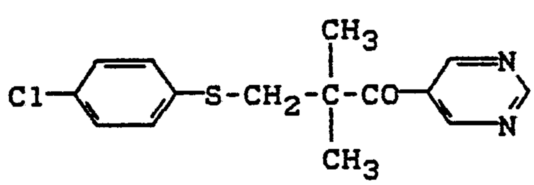 (8) | 0,025 | 16,7 |

## Tabelle A - Fortsetzung

Leptosphaeria nodorum-Test (Weizen)/protektiv

| Wirkstoff | Wirkstoffkonzentration in der Spritzbrühe in Gew.-% | Krankheitsbefall in % der unbehandelten Kontrolle |
|---|---|---|
| 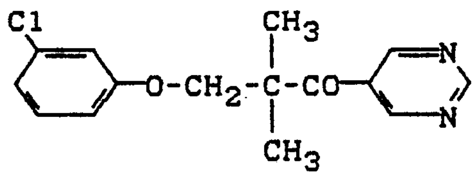 (9) | 0,025 | 33,3 |
| 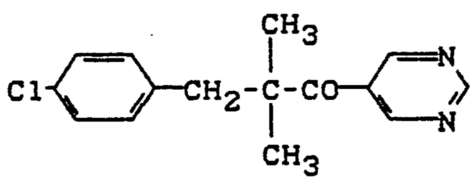 (10) | 0,025 | 0,0 |
| 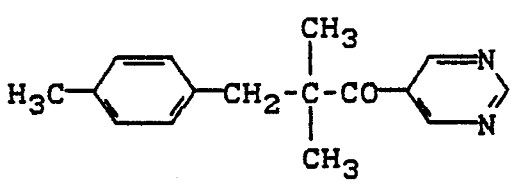 (13) | 0,025 | 7,5 |
| 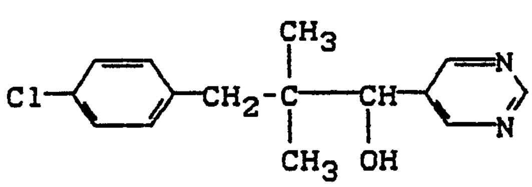 (14) | 0,025 | 8,7 |

Tabelle A - Fortsetzung

Leptosphaeria nodorum-Test (Weizen)/protektiv

| Wirkstoff | Wirkstoffkonzentration in der Spritzbrühe in Gew.-% | Krankheitsbefall in % der unbehandelten Kontrolle |
|---|---|---|
| 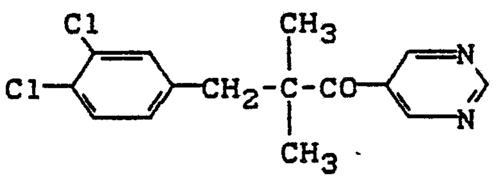 (19) | 0,025 | 0,0 |
| 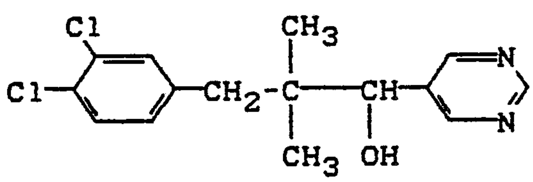 (20) | 0,025 | 8,7 |
| 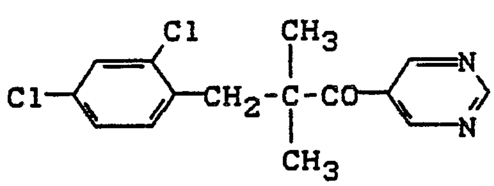 (21) | 0,025 | 0,0 |
| 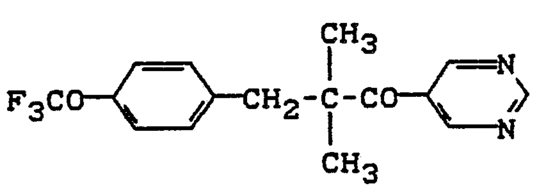 (24) | 0,025 | 0,0 |

## Tabelle A - Fortsetzung

Leptosphaeria nodorum-Test (Weizen)/protektiv

| Wirkstoff | Wirkstoffkonzentration in der Spritzbrühe in Gew.-% | Krankheitsbefall in % der unbehandelten Kontrolle |
|---|---|---|
| 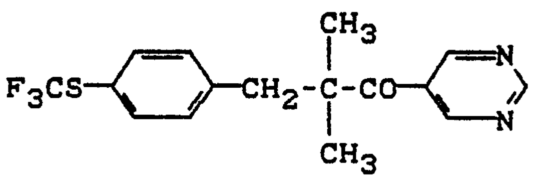 (1) | 0,025 | 3,7 |
| 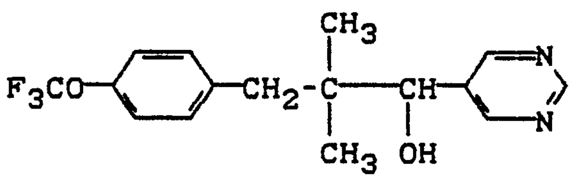 (25) | 0,025 | 0,0 |
| 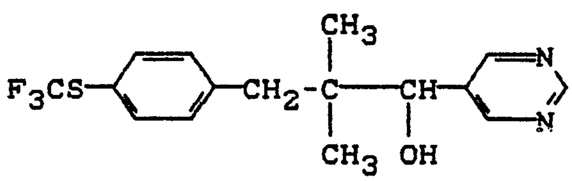 (2) | 0,025 | 0,0 |

**Ansprüche**

1. Schädlingsbekämpfungsmittel, gekennzeichnet durch einen Gehalt an mindestens einem substituierten Pyrimidin der allgemeinen Formel (I),

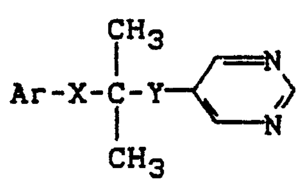 (I)

in welcher
Ar für gegebenenfalls substituiertes Phenyl steht,
X für Sauerstoff, Schwefel, Sulfinyl, Sulfonyl oder eine der Gruppen $-CH_2-$; $-O-CH_2-$; $-CH_2-O-$; $-O-CH_2-CH_2-$; $-S(O)_n-CH_2-$; $-CH_2-S(O)_n-$oder $-S(O)_n-CH_2-CH_2-$steht und
Y für eine der Gruppen

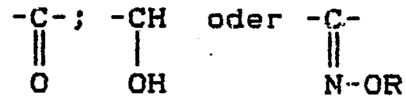

steht, wobei
R für Wasserstoff, Alkyl, Alkenyl, Alkinyl oder für gegebenenfalls substituiertes Aralkyl steht und
n für eine Zahl 0, 1 oder 2 steht,
sowie ihre Säureadditionssalze und Metallsalzkomplexe.

2. Schädlingsbekämpfungsmittel gemäß Anspruch 1, worin in der Formel (I)
Ar für gegebenenfalls einfach bis mehrfach, gleich oder verschieden substituiertes Phenyl steht, wobei als Substituenten infrage kommen: Halogen, Cyano, Nitro sowie jeweils geradkettiges oder verzweigtes Alkyl, Alkoxy, Alkylthio, Alkylsulfinyl oder Alkylsulfonyl mit jeweils 1 bis 4 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 gleichen oder verschiedenene Halogenatomen, jeweils geradkettiges oder verzweigtes Alkoxycarbonyl oder Alkoximinoalkyl mit jeweils 1 bis 4 Kohlenstoffatomen in den einzelnen Alkylteilen, gegebenenfalls einfach bis mehrfach, gleich oder verschieden durch Halogen substituiertes, zweifach verknüpftes Dioxyalkylen oder jeweils gegebenenfalls einfach bis mehrfach, gleich oder verschieden durch Halogen substituiertes Phenyl oder Phenoxy,
X für Sauerstoff, Schwefel, Sulfinyl, Sulfonyl oder für eine der Gruppen $-CH_2-$; $-O-CH_2-$; $-CH_2-O-$; $-O-CH_2-CH_2-$; $-S(O)_n-CH_2-$; $-CH_2-S(O)_n$-oder $-S(O)_n-CH_2-CH_2$-steht und
Y für eine der Gruppen

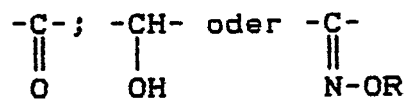

steht, wobei
R für Wasserstoff oder für jeweils geradkettiges oder verzweigtes Alkyl mit 1 bis 6 Kohlenstoffatomen, Alkenyl mit 3 bis 5 Kohlenstoffatomen oder Alkinyl mit 3 bis 5 Kohlenstoffatomen oder für gegebenenfalls einfach bis mehrfach, gleich oder verschieden substituiertes Benzyl oder Phenylethyl steht, wobei als Substituenten im Phenylteil jeweils die bei dem Rest Ar genannten infrage kommen und
n für eine Zahl 0, 1 oder 2 steht sowie ihre Säureadditionssalze und Metallsalzkomplexe.

3. Schädlingsbekämpfungsmittel gemäß Anspruch 1, worin in der Formel (I)
Ar für gegebenenfalls ein-bis dreifach, gleich oder verschieden substituiertes Phenyl steht, wobei als Substituenten infrage kommen: Fluor, Chlor, Brom, Cyano, Nitro, Methyl, Ethyl, n-oder i-Propyl, n-, i-, s-oder t-Butyl, Methoxy, Ethoxy, n-oder i-Propoxy, Methylthio, Ethylthio, Trifluormethyl, Dichlorfluormethyl, Difluorchlormethyl, Difluorbrommethyl, Trichlormethyl, Fluormethyl, Difluormethyl, Trifluorethyl, Tetrafluorethyl, Trifluorchlorethyl, Pentafluorethyl, Trifluormethoxy, Difluormethoxy, Fluormethoxy, Dichlorfluormethoxy, Difluorchlormethoxy, Difluorbrommethoxy, Trichlormethoxy, Trifluorethoxy, Tetrafluorethoxy, Pentafluorethoxy, Trifluorchlorethoxy, Trifluordichlorethoxy, Difluortrichlorethoxy, Pentachlorethoxy, Trifluormethylthio, Difluormethylthio, Fluormethylthio, Difluorchlormethylthio, Dichlorfluormethylthio, Difluorethylthio, Difluorbrommethylthio, Trichlormethylthio, Trifluorethylthio, Tetrafluorethylthio, Pentafluorethylthio, Trifluorchlorethylthio, Trifluordichlorethylthio, Pentachlorethylthio, Methylsulfinyl, Trifluormethylsulfinyl, Dichlorfluormethylsulfinyl, Difluorchlormethylsulfinyl, Fluormethylsulfinyl, Difluormethylsulfinyl, Methylsulfonyl, Trifluormethylsulfonyl, Dichlorfluormethylsulfonyl, Difluorchlormethylsulfonyl, Fluormethylsulfonyl, Difluormethylsulfonyl, Methoxycarbonyl, Ethoxycarbonyl, Methoximinomethyl, Ethoximinomethyl, Methoximinoethyl, Ethoximinoethyl, Difluordioxymethylen, Tetrafluordioxyethylen, Trifluordioxyethylen, Difluordioxyethylen, Dioxymethylen, Dioxyethylen sowie jeweils gegebe nenfalls ein-bis dreifach durch Fluor oder Chlor substituiertes Phenyl oder Phenoxy,
Z für Sauerstoff, Schwefel, Sulfinyl, Sulfonyl oder für eine der Gruppen $-CH_2-$; $-O-CH_2-$; $-CH_2-O-$; $-O-CH_2-CH_2-$; $-S(O)_n-CH_2-$; $-CH_2-S(O)_n$-oder $-S(O)_n-CH_2-CH_2$-steht und
Y für eine der Gruppen

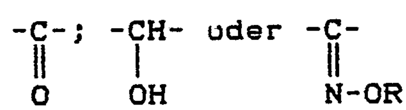

steht, wobei
R für Wasserstoff, Methyl, Ethyl, n-oder i-Propyl, Allyl, Propargyl oder für gegebenenfalls ein-bis dreifach, gleich oder verschieden substituiertes Benzyl steht, wobei als Substituenten die bei dem Rest Ar genannten infrage kommen und
n für eine Zahl 0, 1 oder 2 steht sowie ihre Säureadditionssalze und Metallsalzkomplexe.

4. Verfahren zur Bekämpfung von Schädlingen, dadurch gekennzeichnet, daß man substituierte Pyrimidine der Formel (I) gemäß dem Anspruch 1 auf Schädlinge oder ihren Lebensraum einwirken läßt.

5. Verwendung von substituierten Pyrimidinen der Formel (I) gemäß dem Anspruch 1 zur Bekämpfung von Schädlingen.

6. Verfahren zur Herstellung von Schädlingsbekämpfungsmitteln zur Bekämpfung von Schädlingen, dadurch gekennzeichnet, daß man substituierte Pyrimidine der Formel (I) gemäß dem Anspruch 1 mit Streckmitteln und/oder oberflächenaktiven Mitteln vermischt.

7. Substituierte Pyrimidine der Formel (IA),

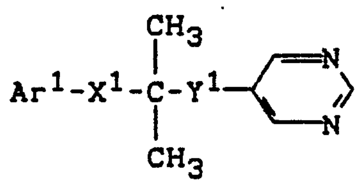

(IA)

in welcher
$Ar^1$ für gegebenenfalls substituiertes Phenyl steht,
$X^1$ für Sauerstoff, Schwefel, Sulfinyl, Sulfonyl oder für eine der Gruppen $-CH_2-$; $-O-CH_2-$; $-CH_2-O-$; $-O-CH_2-CH_2-$; $-S(O)_n-CH_2-$; $-CH_2-S(O)_n-$oder $-S(O)_n-CH_2-CH_2-$steht und
$Y^1$ für eine der Gruppen

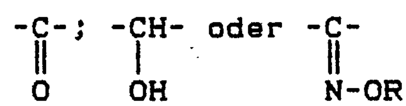

steht, wobei
R für Wasserstoff, Alkyl, Alkenyl, Alkinyl oder für gegebenenfalls substituiertes Aralkyl steht und
n für eine Zahl 0,1 oder 2 steht,
ausgenommen die Verbindungen 2,2-Dimethyl-3-(4-(fluorphenyl)-1-(pyrimidin-5-yl)-propan-1-on, 2,2-Dimethyl-3-(3-chlorphenoxy)-1-(pyrimidin-5-yl)-propan-1-on, 2,2-Dimethyl-2-(4-chlorphenoxy)-1-(pyrimidin-5-yl)-ethan-1-on und 2,2-Dimethyl-3-(4-chlorphenylthio)-1-(pyrimidin-5-yl)-propan-1-on.

8. Substituierte Pyrimidine gemäß Anspruch 7, worin in der Formel (IA)
$Ar^1$ für gegebenenfalls einfach bis mehrfach, gleich oder verschieden substituiertes Phenyl steht, wobei als Substituenten infrage kommen: Halogen, Cyano, Nitro sowie jeweils geradkettiges oder verzweigtes Alkyl, Alkoxy, Alkylthio, Alkylsulfinyl oder Alkylsulfonyl mit jeweils 1 bis 4 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 gleichen oder verschiedenene Halogenatomen, jeweils geradkettiges oder verzweigtes Alkoxycarbonyl oder Alkoximinoalkyl mit jeweils 1 bis 4 Kohlenstoffatomen in den einzelnen Alkylteilen, gegebenenfalls einfach bis mehrfach, gleich oder verschieden durch Halogen substituiertes, zweifach verknüpftes Dioxyalkylen oder jeweils gegebenenfalls einfach bis mehrfach, gleich oder verschieden durch Halogen substituiertes Phenyl oder Phenoxy,
$X^1$ für Sauerstoff, Schwefel, Sulfinyl, Sulfonyl oder für eine der Gruppen $-CH_2-$; $-O-CH_2-$; $-CH_2-O-$; $-O-CH_2-CH_2-$; $-S(O)_n-CH_2-$; $-CH_2-S(O)_n-$oder $-S(O)_n-CH_2-CH_2-$steht und
$Y^1$ für eine der Gruppen

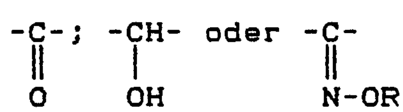

steht, wobei
R für Wasserstoff oder für jeweils geradkettiges oder verzweigtes Alkyl mit 1 bis 6 Kohlenstoffatomen,

Alkenyl mit 3 bis 5 Kohlenstoffatomen oder Alkinyl mit 3 bis 5 Kohlenstoffatomen oder für gegebenenfalls einfach bis mehrfach, gleich oder verschieden substituiertes Benzyl oder Phenylethyl steht, wobei als Substituenten im Phenylteil jeweils die bei dem Rest Ar genannten infrage kommen und
n für eine Zahl 0, 1 oder 2 steht,
ausgenommen die Verbindungen 2,2-Dimethyl-3-(4-fluorphenyl)-1-(pyrimidin-5-yl)-propan-1-on, 2,2-Dimethyl-3-(3-chlorphenoxy)-1-(pyrimidin-5-yl)-propan-1-on, 2,2-Dimethyl-2-(4-chlorphenoxy)-1-(pyrimidin-5-yl)-ethan-1-on und 2,2-Dimethyl-3-(4-chlorphenylthio)-1-(pyrimidin-5-yl)-propan-1-on.

9. Substituierte Pyrimidine gemäß Anspruch 7, worin in der Formel (IA)
$Ar^1$ für gegebenenfalls ein-bis dreifach, gleich oder verschieden substituiertes Phenyl steht, wobei als Substituenten infrage kommen: Fluor, Chlor, Brom, Cyano, Nitro, Methyl, Ethyl, n-oder i-Propyl, n-, i-, s-oder t-Butyl, Methoxy, Ethoxy, n-oder i-Propoxy, Methylthio, Ethylthio, Trifluormethyl, Dichlorfluormethyl, Difluorchlormethyl, Difluorbrommethyl, Trichlormethyl, Fluormethyl, Difluormethyl, Trifluorethyl, Tetrafluorethyl, Trifluorchlorethyl, Pentafluorethyl, Trifluormethoxy, Di fluormethoxy, Fluormethoxy, Dichlorfluormethoxy, Difluorchlormethoxy, Difluorbrommethoxy, Trichlormethoxy, Trifluorethoxy, Tetrafluorethoxy, Pentafluorethoxy, Trifluorchlorethoxy, Trifluordichlorethoxy, Difluortrichlorethoxy, Pentachlorethoxy, Trifluormethylthio, Difluormethylthio, Fluormethylthio, Difluorchlormethylthio, Dichlorfluormethylthio, Difluorbrommethylthio, Trichlormethylthio, Trifluorethylthio, Difluorethylthio, Tetrafluorethylthio, Pentafluorethylthio, Trifluorchlorethylthio, Trifluordichlorethylthio, Pentachlorethylthio, Methylsulfinyl, Trifluormethylsulfinyl, Dichlorfluormethylsulfinyl, Difluorchlormethylsulfinyl, Fluormethylsulfinyl, Difluormethylsulfinyl, Methylsulfonyl, Trifluormethylsulfonyl, Dichlorfluormethylsulfonyl, Difluorchlormethylsulfonyl, Fluormethylsulfonyl, Difluormethylsulfonyl, Methoxycarbonyl, Ethoxycarbonyl, Methoximinomethyl, Ethoximinomethyl, Methoximinoethyl, Ethoximinoethyl, Difluordioxymethylen, Tetrafluordioxyethylen, Trifluordioxyethylen, Difluordioxyethylen, Dioxymethylen, Dioxyethylen sowie jeweils gegebenenfalls ein-bis dreifach durch Fluor oder Chlor substituiertes Phenyl oder Phenoxy,
$X^1$ für Sauerstoff, Schwefel, Sulfinyl, Sulfonyl oder für eine der Gruppen $-CH_2-$; $-O-CH_2-$; $-CH_2-O-$; $-O-CH_2-CH_2-$; $-S(O)_n-CH_2-$; $-CH_2-S(O)_n-$oder $-S(O)_n-CH_2-CH_2-$steht und
$Y^1$ für eine der Gruppen

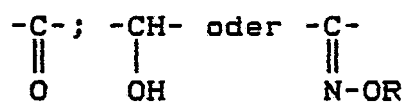

steht wobei
R für Wasserstoff, Methyl, Ethyl, n-oder i-Propyl, Allyl, Propargyl oder für gegebenenfalls ein-bis dreifach, gleich oder verschieden substituiertes Benzyl steht, wobei als Substituenten die bei dem Rest Ar genannten infrage kommen und
n für eine Zahl 0, 1 oder 2 steht,
ausgenommen die Verbindungen 2,2-Dimethyl-3-(4-fluorphenyl)-1-(pyrimidin-5-yl)-propan-1-on, 2,2-Dimethyl-3-(3-chlorphenoxy)-1-(pyrimidin-5-yl)-propan-1-on, 2,2-Dimethyl-2-(4-chlorphenoxy)-1-(pyrimidin-5-yl)-ethan-1-on und 2,2-Dimethyl-3-(4-chlorphenylthio)-1-(pyrimidin-5-yl)-propan-1-on.

10. Verfahren zur Herstellung von substituierten Pyrimidinen der Formel (IA)

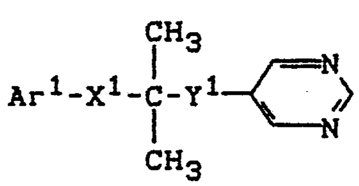

(IA)

in welcher
$Ar^1$ für gegebenenfalls substituiertes Phenyl steht,
$X^1$ für Sauerstoff, Schwefel, Sulfinyl, Sulfonyl oder für eine der Gruppen $-CH_2-$; $-O-CH_2-$; $-CH_2-O-$; $-O-CH_2-CH_2-$; $-S(O)_n-CH_2-$; $-CH_2-S(O)_n-$oder $-S(O)_n-CH_2-CH_2-$steht und
$Y^1$ für eine der Gruppen

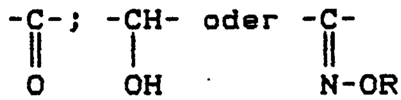

steht, wobei

R für Wasserstoff, Alkyl, Alkenyl, Alkinyl oder für gegebenenfalls substituiertes Aralkyl steht und

n für eine Zahl 0,1 oder 2 steht,

ausgenommen die Verbindungen 2,2-Dimethyl-3-(4-fluorphenyl)-1-(pyrimidin-5-yl)-propan-1-on, 2,2-Dimethyl-3-(3-chlorphenoxy)-1-(pyrimidin-5-yl)-propan-1-on, 2,2-Dimethyl-2-(4-chlorphenoxy)-1-(pyrimidin-5-yl)-ethan-1-on und 2,2-Dimethyl-3-(4-chlorphenylthio)-1-(pyrimidin-5-yl)-propan-1-on, dadurch gekennzeichnet, daß man

(a) für den Fall, daß $Y^1$ für - 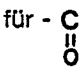 -steht, die substituierten Pyrimidine der Formel (Ia),

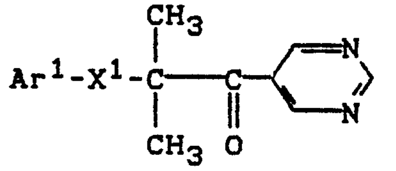 (Ia)

in welcher

$Ar^1$ und $X^1$ die oben angegebene Bedeutung haben,

erhält,

wenn man Acylenole der Formel (II),

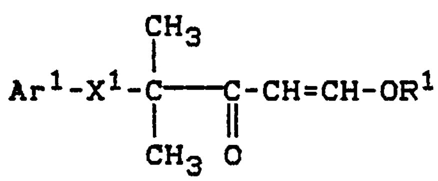 (II)

in welcher

$Ar^1$ und $X^1$ die oben angegebene Bedeutung haben und

$R^1$ für Wasserstoff oder ein Alkalimetallkation steht,

nacheinander mit Formamidin oder einem Säureadditionssalz des Formamidins und dann mit Formaldehyddimethylacetal gegebenenfalls in Gegenwart eines Verdünnungsmittels umsetzt und

(b) für den Fall, daß $Y^1$ für - 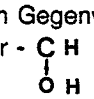 -steht,

die substituierten Pyrimidine der Formel (Ib),

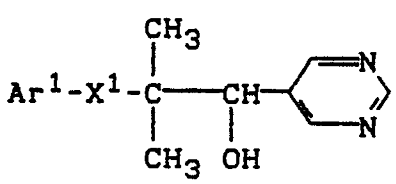 (Ib)

in welcher

$Ar^1$ und $X^1$ die oben angegebene Bedeutung haben,

erhält,

wenn man die nach Verfahren (a) oder (d) erhältlichen substituierten Pyrimidine der Formel (Ia),

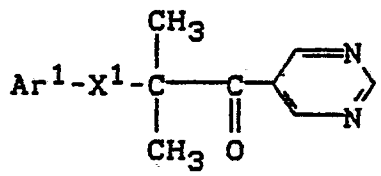 (Ia)

in welcher
$Ar^1$ und $X^1$ die oben angegebene Bedeutung haben,
mit einem Reduktionsmittel gegebenenfalls in Gegenwart eines Verdünnungsmittels umsetzt;

(c) für den Fall, daß $Y^1$ für - C -steht,
‖
NOR

die substituierten Pyrimidine der Formel (Ic),

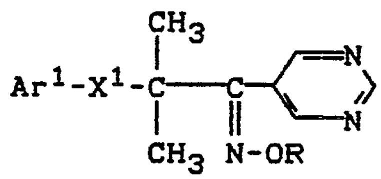 (Ic)

in welcher
$Ar^1$, $X^1$ und R die oben angegebene Bedeutung haben,
erhält,
wenn man die nach Verfahren (a) oder (d) erhältlichen substituierten Pyrimidine der Formel (Ia),

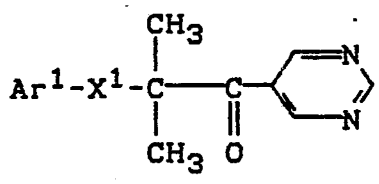 (Ia)

in welcher
$Ar^1$ und $X^1$ die oben angegebene Bedeutung haben,
mit Hydroxylaminen der Formel (III),

$R\text{-}O\text{-}NH_2$ (III)

in welcher
R die oben angegebene Bedeutung hat,
oder mit deren Säureadditionssalzen gegebenenfalls in Gegenwart eines Verdünnungsmittels und gegebenenfalls in Gegenwart eines Säurebindemittels umsetzt und

(d) man substituierte Pyrimidine der Formel (Id),

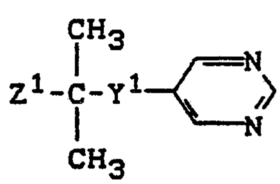 (Id)

in welcher
$Z^1$ für einen Rest $Ar^1$-$X^2$-oder für einen Rest $Ar^2$-$X^1$-steht, wobei
$Ar^1$, $X^1$ und $Y^1$ die oben angegebene Bedeutung haben,
$Ar^2$ für einen durch Alkylsulfinyl, Alkylsulfonyl, Halogenalkylsulfinyl oder Halogenalkylsulfonyl substituierten Phenylrest steht,
$X^2$ für Sulfinyl, Sulfonyl oder eine der Gruppen $-S(O)_m-CH_2-$; $-CH_2-S(O)_m-$oder $-S(O)_m-CH_2-CH_2-$steht und
m für eine Zahl 1 oder 2 steht, erhält,
wenn man die nach Verfahren (a), (b) oder (c) erhältlichen substituierten Pyrimidine der Formel (Ie),

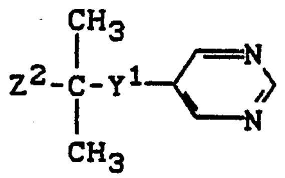 (Ie)

in welcher
$Z^2$ für einen Rest $Ar^1$-$X^3$-oder für einen Rest $Ar^3$-$X^1$-steht, wobei
$Ar^1$, $X^1$ und $Y^1$ die oben angegebene Bedeutung haben,
$Ar^3$ für einen durch Alkylthio oder Halogenalkylthio substituierten Phenylrest steht und
$X^3$ für Schwefel oder eine der Gruppen $-S-CH_2-$; $-CH_2-S-$oder $-S-CH_2-CH_2-$steht,
mit einem Oxidationsmittel, gegebenenfalls in Gegenwart eines Verdünnungsmittels und gegebenenfalls in Gegenwart eines Reaktionshilfsmittels umsetzt, und gegebenenfalls an die mit Hilfe der Herstellungsverfahren (a), (b), (c) oder (d) erhältlichen Verbindungen eine Säure oder ein Metallsalz addiert.